(19)

Europäisches
Patentamt
European
Patent Office
Office européen
des brevets

(11) **EP 4 239 642 A1**

(12) **EUROPEAN PATENT APPLICATION**

(43) Date of publication:
**06.09.2023 Bulletin 2023/36**

(21) Application number: **22159795.8**

(22) Date of filing: **02.03.2022**

(51) International Patent Classification (IPC):
**G16H 15/00** (2018.01)  **G06F 40/00** (2020.01)
**G06T 7/00** (2017.01)  **G16H 50/20** (2018.01)

(52) Cooperative Patent Classification (CPC):
**G16H 15/00; G06F 40/30; G06T 7/0012;
G16H 50/20;** G06F 40/205; G06F 40/284;
G06F 40/295

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Siemens Healthcare GmbH
91052 Erlangen (DE)**

(72) Inventor: **Conjeti, Sailesh
91056 Erlangen (DE)**

Remarks:
Amended claims in accordance with Rule 137(2)
EPC.

(54) **METHOD FOR GENERATING PROTOCOL DATA OF A RADIOLOGICAL IMAGE DATA MEASUREMENT**

(57)     A method for generating protocol data (PD, MDO3, CDS, PRR) of a specific medical process (SMP) is described. The specific medical process (SMP) comprises at least one measurement process, preferably a radiological imaging process. The method comprises the steps of generating a first structured medical data object (MDO1, UDS, KG) based on user-generated electronic medical data (UMD, TMD, RR, VEMD, AVEMD). The user-generated electronic medical data (UMD, TMD, RR, VEMD, AVEMD) are created by an expert documenting the specific medical process (SMP). Further, the method includes the step of generating a second structured medical data object (MDO2, ADS, AI-KG) by applying an automatic processing algorithm (APA, AIM, MC) to automatically created electronic medical data (AMD, RI). The electronic medical data (AMD, RI) refer to the specific medical process (SMP). Furthermore, the method comprises the step of generating the protocol data (PD, MDO3, CDS, PRR) based on the first structured medical data object (MDO1, UDS, KG) and on the second structured medical data object (MDO2, ADS, AI-KG), wherein the first and second structured medical data objects (MDO1, UDS, KG, MDO2, ADS, AI-KG) are compared to determine deviations (DV). Moreover, a protocol data generation device (60) is described. Furthermore, a medical process system (1) is described.

FIG 1

EP 4 239 642 A1

**Description**

The invention relates to a method for generating protocol data of a specific medical process. The invention also concerns a protocol data generation device. Further, the invention relates to a medical process system.

Radiology reporting is typically done by a qualified medical professional via dictation and free-text entry in the native reporting language. Best practice as proposed by the Radiological Society of North America Radiology Reporting Committee includes that a radiologist includes the following sections as part of a radiology report: patient identifiers, imaging procedure description, imaging findings and summary information. This is disclosed in Kahn Jr, C.E. et al, 2009. Toward best practices in radiology reporting. Radiology, 252(3), pp. 852-856.

In a survey by Powell and Silberzweig on the state of structured reporting in the USA nearly 51.3 % of radiologists come from groups that developed structured reporting for at least half of their reports. The survey is disclosed in Powel, D.K. and Silberzweig, J.E., 2015. State of structured reporting in radiology, a survey. Academic radiology, 22(2), pp. 226-233. This trend is on the raise with position papers from the ESR (ESR is an acronym for European Society of Radiology) and other radiological societies advocating for use of standardized terminology and reporting templates for radiology reporting over prose text. Such position papers are disclosed in European Society of Radiology (ESR) communications@myesr. org, 2018. ESR paper on structured reporting in radiology. Insights into imaging, 9, pp. 1-7.

For supporting adoption of structured reporting in radiology, healthcare companies provide products that are capable of automated measurements and characterization of the measurements based on artificial intelligence (abbreviated by the acronym AI). The results are provided in form of DICOM Structured Reporting objects (abbreviated as DICOM SR objects, DICOM is an acronym for an open standard for storing and exchanging information in medical image data management named "Digital Imaging and Communications in Medicine") with appropriately mapped concept groups. However, the reports provided in the form of DICOM SR objects are typically static reports with often limited predefined measurements and are not updated automatically after a radiologist has finished reporting on a case. In case a radiologist re-measures a particular measurement or performs a new measurement, the DICOM SR object is not updated to reflect this. Hence, we now have a static DICOM SR object and a free-text radiology report or annotations or markups in XML that have not been reconciled. This gap is further amplified if the dictation language for the free-text radiology report is not English, which is typically the encoding language for DICOM SR objects.

A DICOM SR object is typically a static object that is generated by an algorithm. Certain PACS (PACS is an acronym for Picture Archiving and Communication System) offer solutions to manually edit the DICOM SR objects. However, there are no publicly disclosed solutions that harmonize later described concept items extracted from free-text radiology reports against AI-generated DICOM SR objects to create and composite DICOM SR objects.

Another aspect of radiology reports is concerned to a quality control of these reports by peer review. Continuous and systematic peer review is an essential means to assess clinical radiologist's performance of diagnostic accuracy and to ensure adherence to a practice of quality improvement within a radiology department. In the current practice, a tool such as RADPEER is used for peer review. RADPEER is a program that allows peer review to be performed during routine image interpretation. After submission of practice data by the radiologist, a responsible person, for example the group chairman or medical director, can access the reports online at any time. RADPEER is advocated in the United States and comprises various steps including case identification, assignment to a reviewing radiologist, peer review, committee review comparing findings from original interpreting radiologist, decision making and communication of findings followed by an improvement plan. Owing to increased workload, radiologist shortage, decrease in reimbursement for reading services etc., this has led to a decreased compliance to peer review systems. The interpersonal element that peer review could have potentially negative influence on relationship between colleagues can also lead to negative attitude towards the current system. This offers opportunities for improving this process with the aid of artificial intelligence. Artificial intelligence can be used adjunct to the current peer review process by analyzing images and radiology reports and serving in real-time as a radiology companion. For such a solution to be successful, explainability of the differences to the interpreting radiologist is the key.

In current radiology workflows, peer review has mainly been done through random selection of cases for review by a peer radiologist and subsequent assessment by a committee. As per the RADPEER system advocated by the American College of Radiology, only cases scored 3 and 4 are discussed further by the committee. Scores 3 and 4 include clinically significant discrepancy of a diagnosis. This practice has led to a selection bias and an under-reporting of serious discrepancies to avoid potential punitive response and due to insufficient statistical power as a large number of samples are needed to find useful discrepancies. Alternatively, solutions such as double reading, professional auditing by comparing against pathology reports, follow-up scans etc., or using focused practice review are used to reduce failure in clinical practice. The potential of using AI for radiology peer review was introduced recently by Rao et al., "Utility of artificial intelligence tool as a prospective radiology peer reviewer - Detection of unreported intracranial hemorrhage", academic radiology, 28(1), pp. 85-93, for the task of using AI to detect unreported intracranial hemorrhages. Mahajan et al., "Deploying deep learning for quality control: An AI-assisted review of chest X-rays reported as normal in routine clinical practice", Radiological Society of North America 2019 Scientific Assembly and Annual Meeting, December 1-6, 2019, Chicago, IL. archive.rsna.org/2019/19008598.html, proposed to use results from a multi-class classifier to select cases for peer review.

However, there is no proposed system for explainable AI-based peer review. The conventional systems only consider simple heuristics-based rules. Such a rule recommends to short-list a case for peer review based on the question if a finding has been concurrently detected or missed by AI.

Hence, there is a problem of using expert knowledge and automatic analytic tools for the analysis of medical measurement data in a more coherent way.

The before-mentioned problem is solved by a method for generating protocol data of a specific medical process according to claim 1, by a protocol data generation device according to claim 12 and by a medical process system according to claim 13.

In the method for generating protocol data of a specific medical process according to the invention, the specific medical process comprises at least one measurement process, preferably a radiological imaging process. A specific medical process includes a medical treatment of a patient. Such a medical treatment includes actions of an expert for prevention, detection and treatment of diseases. Protocol data include information, for example measurement data, description data or protocol parameter, which characterize, for example describe or determine the specific medical process. These protocol data can include a report describing the specific medical process or control data, for example control parameter data, for predetermining the specific medical process and in particular the at least one measurement process. However, it has to be mentioned that a preferred field of use of the method according to the invention is the use of protocol data for reporting and recording measurement data, in particular radiological imaging data, of a specific medical process. A measurement process is an experimental process for determining a specific value of a physical size as a multiple of a unit or a reference value as a measurement value. In particular, such a measurement process includes all kinds of medical imaging processes, especially radiological imaging processes. A measurement process is performed using technical means, in particular measuring devices, most notably imaging devices, for generating measurement values.

The method according to the invention comprises the following steps: a first structured medical data object is generated based on user-generated electronic medical data. These user-generated electronic medical data are generated by a user, in particular an expert, who preferably documents or prepares the specific medical process. The first structured medical data object can be generated by extracting data elements from the user-generated electronic medical data. In case the user-generated electronic medical data include free-text, later described text-analysis methods including parsing processes and a method of determining concept items representing entities in the free-text and determining relationships between the found concept items can be used. Concept items include name/value pairs characterizing an identified entity.

Principles of parsing texts are described in Pyysalo S. (2013) "Text Parsing", in Dubitzky W., Wolkenhauer O., Cho KH., Yokota H. (eds) Encyclopedia of Systems Biology, Springer, New York, NY, https://doi.org/10.1007/978-1-4419-9863-7_182.

Further, also a second structured medical data object is generated by applying an automatic processing algorithm to automatically created electronic medical data. The automatically created electronic medical data refer to the specific medical process. "Automatically created" means that these data are not user-generated. In contrast thereto, they are generated by using an automatic process, in particular an imaging process or another kind of measurement process. An automatic processing algorithm is performed without intervention of a user. Preferably, an automatic processing algorithm includes a model based on artificial intelligence. Typically, the automatic processing algorithm performs a task which is usually or alternatively carried out by an expert without intervention of an expert. Such a task can comprise an examination including an annotation and/or finding based on the automatically created electronic medical data. AI-based annotation and finding in image data is described in the following documents:

> Tang, Y.X., Tang, Y.B., Peng, Y., Yan, K., Bagheri, M., Redd, B.A., Brandon, C.J., Lu, Z., Han, M., Xiao, J. and Summers, R.M., 2020. Automated abnormality classification of chest radiographs using deep convolutional neural networks. NPJ digital medicine, 3(1), pp.1-8 and

> Calli, E., Sogancioglu, E., van Ginneken, B., van Leeuwen, K.G. and Murphy, K., 2021. Deep learning for chest X-ray analysis: A survey. Medical Image Analysis, 72, p.102125.

Further, the results of the automatic processing are transformed to the formal structure of the second structured medical data object. That can be achieved by organizing the results in a hierarchical fashion of concept items. In case the second structured medical data object comprises a predefined structure, a particular template can be used for generating the predefined structure as later described. As later described in detail, in a particular variant, the predefined structure can include the structure of a hierarchical graph, in particular a DICOM SR object. An automatic examination of radiology image data and a transformation of the results of the examination, i.e. the automatic processing, into the structure of a DICOM SR object is described in the following documents:

Editor: Kevin O'Donnell

Contributors: DICOM WG-23, David Clunie, Eliot Siegel, Julian Marshal,

URL:
https://wiki.ihe.net/index.php/AI_Results_-_Brief_Proposal, access on 12.02.2022 and

Radiology Technical Committee, IHE Radiology Technical Framework Supplement, AI Results (AIR) 15 Revision 1.1 - Trial Implementation, July 16, 2020,

URL:
https://www.ihe.net/uploadedFiles/Documents/Radiology/IHE_RAD _Suppl_AIR.pdf.

If the second structured medical data object is based on a dynamic graph structure, the second structured medical data object can be generated based on a global graph structure and by reducing and refining the global graph structure, preferably by extracting a number of key features using computer aided detection or diagnostic tools as described later.

Key features are radiological findings associated with a certain body part or modality. They are usually domain knowledge and can be found in glossaries such as the following:

Hansell, D.M., Bankier, A.A., MacMahon, H., McLoud, T.C., Muller, N.L. and Remy, J., 2008. Fleischner Society: glossary of terms for thoracic imaging. Radiology, 246(3), pp.697-722,

Palmer, W., Bancroft, L., Bonar, F., Choi, J.A., Cotten, A., Griffith, J.F., Robinson, P. and Pfirrmann, C.W., 2020. Glossary of terms for musculoskeletal radiology. Skeletal Radiology, 49(1), pp.1-33 and

Atalar, M., Öztoprak, B., Gümüs, C., Egilmez, H. and Bulut, S., 2014, March. Classical Signs and Appearances in Pediatric Neuroradiology. European Congress of Radiology-ECR 2014.

The above-mentioned automatically created electronic medical data include measurement data. These measurement data are generated or recorded by a measuring system, for example by a radiological imaging system. In contrast to the user-generated electronic medical data, automatically created electronic medical data are directly available in an automatically processable form. In other words, the user-generated electronic medical data must first be brought into the form of a structured medical data object to be after that automatically processable. In contrast thereto, the automatically created electronic medical data are already in an automatically processable form.

Structured medical data objects comprise medical data elements that are arranged according to defined structuring rules in a defined, predetermined structure. Such a structure can be the structure of an ontology or a data base structure, in particular a table.
An ontology encompasses a linguistically composed and formally ordered representation of a set of concepts and the relationships between them in a domain, i.e. a specific subject area. The description of common properties in an ontology is defined as a concept. Concepts are also referred to as classes. Types represent object types in the ontology and represent the available types in the classes. Instances represent objects or entities in the ontology. They are generated using previously defined terms and are also referred to as individuals. Entities can be described by concept items, i.e. name/value-pairs.
In contrast thereto, as already mentioned-above, the user-generated electronic medical data are typically not generated in such a formal structure of a structured medical data object. As later described in detail, the user-generated electronic medical data may include unprocessed measurement data and data generated by a human person, preferably free-text data. Therefore, these user-generated electronic medical data have to be brought into a formal structure by extraction and transformation.
Based on the first structured medical data object and on the second structured medical data object, protocol data are generated. The generation of protocol data is implemented by comparing the first and second structured medical data objects to determine deviations, in particular contradictory differences or complements. Deviations include deviations, i.e. contradictory or non-contradictory differences between the first structured medical data object and the second structured medical data object. Complements refer to data differences between the first and the second structured medical data object that are not contradictory.
It has to be mentioned that the method according to the invention can also be applied to more than two structured medical data objects. For example, it may be possible that two different second structured medical data objects are generated using the same or different automatic processing algorithms and both structured medical data objects are reconciled with the first structured medical data object.
To bridge the gap between automatically, for example by artificial intelligence, generated structured medical data objects and conventionally generated informal data, i.e. user-generated electronic medical data, preferably generated by human reporting, the proposed method according to the invention utilizes automatic extraction techniques for finding data elements in the user-generated electronic medical data which can be automatically processed and examined. As later discussed in detail, such extraction techniques can comprise Natural Language Processing to parse the user-generated electronic medical data, for example a free-text DICOM report. Then, the parsed data elements are mapped to standardized semantic concepts used for encoding the first structured medical data object along with the right encoding concept items and relationships between them.

The first structured medical data object extracted from the user-generated documentation of the specific medical process, i.e. the user-generated electronic medical data, is then compared and contrasted with the second structured medical data object, for example an AI-generated structured medical data object. As later described, the step of comparison and contrasting can be implemented as a merging process. A merging process is a process of reconciling multiple changes made to different versions of a data set. In this context, the first and second structured medical data object may be interpreted as different versions concerned to the same content, i.e. the same specific medical process.

If there is a discrepancy found, the automatic measurement, for example annotated by an artificial intelligence, needs to be reconciled with human measurement and findings or vice versa. The merged structured medical data objects form a final verified protocol data, preferably including a final verified third structured medical data object that can then be committed to an archive or a data file, for example to an electronic health record of the patient related to the specific medical process. The described workflow is preferably fully automated and preferably happens in the background as a particular case is being examined, preferably reported, and does not create additional burden to the user.

The method according to the invention can be used for validating medical analysis data, in particular annotation data or finding data, generated by using an automatic processing algorithm. The validation is performed additionally based on user-generated data which may enjoy a higher level of trust than automatically generated data. Vice versa, the method according to the invention can also be used for validating user-generated data based on additional automatically generated medical analysis data, in particular finding data. Such a process can be named as an automatic peer review. Advantageously, in both cases, the result is more trustable compared to the case which only includes one single data source for a medical analysis, in particular a finding or annotation process. Further, in case of an automatic peer review, the user is spared the uncomfortable feeling of being checked by another human expert. Hence, personal conflicts between colleagues resulting from a different assessment of medical data can be avoided. Advantageously, the method is accommodated for potential variations in reporting styles and a learningbased approach is used to decide if a discrepancy needs to be remarked upon by the user.

The protocol data generation device according to the invention comprises an extraction unit for generating a first structured medical data object based on user-generated electronic medical data, which user-generated electronic medical data are created by a user, preferably an expert, documenting a specific medical process comprising at least one measurement process.

Part of the protocol data generation device according to the invention is also a processing unit for generating a second structured medical data object by applying an automatic processing algorithm to automatically created electronic medical data, which electronic medical data refer to the specific medical process.

The protocol data generation device according to the invention comprises also a data generation unit for generating protocol data based on the first structured medical data object and on the second structured medical data object, wherein the first and second structured medical data object are compared to determine deviations. The protocol data generation device according to the invention shares the advantages of the method for generating protocol data of a specific medical process according to the invention.

The medical process system, preferably a medical imaging system, according to the invention comprises a process unit for applying a specific medical process to a patient. Such a process unit applies a medical process, which includes at least one measurement process, to a patient. Preferably, the measurement process includes a medical imaging process, i.e. a radiological process. Preferably, the process unit includes a scan unit for recording raw data from the patient for performing a medical imaging process.

Further, the medical process system includes a documentation unit for generating automatically created electronic medical data documenting the specific medical process. Preferably, the documentation unit includes means for processing measurement data of the specific medical process, particularly preferably, the means are used for generating medical image data. Preferably, the documentation unit includes a reconstruction unit for generating imaging data based on raw data received from the process unit, in that special variant a scan unit.

Furthermore, the medical process system includes a user interface for receiving user-generated electronic medical data created by a user documenting the specific medical process. As later described in detail, the user-generated electronic medical data can include a medical report, preferably a radiological report in case image data are processed.

The medical process system according to the invention also comprises a protocol data generation device according to the invention. As mentioned above, the protocol data generation device generates a first structured medical data object based on the user-generated electronic medical data and a second structured medical data object based on the automatically created electronic medical data. The first and the second structured medical data object are compared to determine deviations between the first and second structured medical data object. The medical process system according to the invention shares the advantages of the method for generating protocol data of a specific medical process according to the invention.

Some units or modules of the protocol data generation device according to the invention, in particular the extraction unit, the processing unit and the data generation unit and some units or modules of the medical process system according to the invention, in particular the process unit, the documentation unit and parts of the user interface, can be completely or partially realized as software modules running on a processor of a respective computing system, e.g. of a control device of a medical process system, in particular a medical imaging system or a medical finding system.

A realization largely in the form of software modules can have the advantage that applications already installed on an existing computing system can be updated with relatively little effort to install and run these units of the present application. The object of the invention is also achieved by a computer program product with a computer program that is directly loadable into the memory of a computing system. Such a computer program comprises program units to perform the steps of the inventive method, at least those steps that could be executed by a computer, especially steps of generating a first structured medical data object, generating a second structured medical data object and generating the protocol data, when the program is executed by the computing system. In addition to the computer program, such a computer program product can also comprise further parts such as a documentation and/or additional components, also hardware components such as a hardware key (dongle etc.) to facilitate access to the software.

A largely software-based implementation has the advantage that medical process systems that have already been used, in particular medical imaging systems, can easily be retrofitted by a software update in order to work in the manner according to the invention.

A computer readable medium such as a memory stick, a harddisk or other transportable or permanently-installed carrier can serve to transport and/or to store the executable parts of the computer program product so that these can be read from a processor unit of a computing system. A processor unit can comprise one or more microprocessors or their equivalents.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method for generating protocol data of a specific medical process according to the invention, a deviation detection reaction is performed if deviations, in particular contradictory differences and/or complements are determined. Such a deviation detection reaction is realized using technical means such as electronic means and/or otherwise physically effective means. Advantageously, an adaption to a detected deviation is enabled to influence, preferably to improve a medical workflow based on the generated protocol data.

Preferably, a deviation detection reaction comprises at least one of the following actions if deviations are determined:

- generating an alert output for a user,
- generating amended protocol data,
- generating protocol data including marks indicating deviations,
- generating protocol data including proposals for correcting the user-generated electronic medical data,
- including measurement data with marked deviations into the protocol data.

Advantageously, a user can be made aware by the deviation detection reaction, in particular by an alert and/or by amended protocol data about the detected deviations. Preferably, the amended protocol data are displayed to the user and the user is such enabled to take note of the amendments or even to react to the amendments. Amendments can include replacements or added data. In case the added data include marks indicating deviations in the protocol data, the user is even directed to the problematic passages in the protocol. In case of including proposals the user even gets an explicit hint how to amend the protocol data for a correction in case the deviations comprise mistakes. Further, the amended protocol data can be saved in an electronic health database, wherein they replace or complement an older version of the protocol data such that a corrected set of protocol data is provided for the user.

In a specific variant, for generating amended protocol data, at least parts of the second structured medical data object, in particular a DICOM SR object, being contradictory to corresponding parts of the first structured medical data object, referring to user-generated medical data, are replaced by parts of the first structured medical data object. In this variant, which is applicable for validating automatically generated second structured medical data objects by user-generated medical data, user-generated results have a higher level of trustworthiness than automatically generated results. However, in this variant, complements of the first structured medical data object which are not contradictory to corresponding data elements of the second structured medical data object are added to the corresponding data elements of the second structured medical data object.

In the variant of "peer review" of the first structured medical data object, for generating protocol data, the first structured medical data object is complemented by the second structured medical data object also in case deviations between the first structured medical data object and the second structured medical data object are contradictory. Then the resulting protocol data are displayed to the user by marking the additional potentially contradictory data. Hence, the user is enabled to decide himself about an amendment of the first structured medical data object based on the received protocol data.

Advantageously, the automatic validation is used to automatically improve the quality of protocol data or to facilitate such a correction or complementation of these protocol data by the user.

Furthermore, the resulting protocol data representing the potentially contradictory data can be optionally converted into human readable clinical text, in particular a text of a radiology report. The conversation can be implemented using a rule based method using templates with named entities as placeholders. An alternative to rule based methods is to employ deep learning models such with a graph convolution encoder feeding to a text generation recurrent neural network-based decoder. Advantageously, a human expert can easily understand the results, i.e. the resulting protocol data without the knowledge of the definition of formalized data structures.

In a further variant of the method for generating protocol data of a specific medical process according to the invention, the generated protocol data comprise a third structured medical data object. Advantageously, at least a part of the protocol data can be processed automatically if intended. In case the type of the third structured medical data object is a standardized data object, this information can be processed further by the usual data processing systems of a medical facility and can also be saved in the existing archives.

In another variant of the method according to the invention the first and the second structured medical data object comprise the same data structure. Advantageously, the first and the second structured medical data object can be directly compared or merged with each other due to an analogue data structure without transforming data of the first structured medical data object into the form of the second structured medical data object or vice versa.

Preferably, the specific medical process comprises at least one of:

- a medical imaging process,
- an electrode based measurement on a patient,
- a lab-diagnostics examination based on a sample of a patient,
- a clinical examination,
- a surgery,
- a clinical discharge process,
- a pathology examination,
- a tumor-boarding,
- a therapy planning,
- a patient monitoring.

Medical imaging is the technique and process of imaging the exterior or interior of a body for clinical analysis and medical intervention, as well as visual representation of the function of some organs or tissues. In this variant, the user-generated electronic medical data document and analyse medical images and the automatically created electronic medical data include the medical images. Hence, in this case, the medical images are concurrently basis for the first structured medical data object and the second structured medical data object. Advantageously, a human-based and an automatic examination of medical images is combined, such that a higher trustworthiness of the examination is achieved compared to conventional examination of images by only one single instance, i.e. either by a human expert alone or by an automatic processing algorithm alone.

An electrode based measurement on a patient uses electrode sensors for measurement of electric currents flowing through the patient due to physiological functions of the body of the patient. For example, an electrode based measurement includes an electrocardiogram or an electroencephalogram. The user-generated electronic medical data include a description of the medical state of the patient by a doctor and the automatically created electronic medical data include the measurement date received from the electrode sensors.

A lab-diagnostics examination based on a sample of a patient includes different types of medical diagnostics, as for example laboratory medicine, bacteriologic or parasitological diagnostics, virus diagnostics, human genetic lab-diagnostics, transfusion medicine lab-diagnostics and toxicological lab-diagnostics. In that variant, the user-generated electronic medical data include a description of the diagnostic result of the patient generated by a doctor and the automatically created electronic medical data include the measurement values of the result of the lab-diagnostics examination.

A clinical examination by a doctor can be directly documented by the doctor. However, in case physiological data are measured, these measurement data can also be directly processed by automatic processing algorithms. Advantageously, the documentation of the human expert can be supplemented by the result of an automatic processing algorithm. Using the method according to the invention, quality and reliability of the results of the clinical examination can be improved.

Also a documentation of a surgery can be performed directly by the surgeon and also based on stored monitoring data automatically recorded by a monitoring system in the operating room. These monitoring data can also be directly processed by automatic processing algorithms. Advantageously, using the method according to the invention, the documentation of the human expert, i.e. the surgeon, can be supplemented by the result of an automatic processing algorithm.

A documentation of a clinical discharge process may be performed based on a free-text document and additionally based on automatically recorded measurement data which justify the discharge of a patient. Advantageously, using the method according to the invention, a documentation of a discharge process by a human expert can be supplemented by the result of an automatic processing algorithm.

Also a pathology examination may include user-generated electronic medical data, in particular a free-text documentation, and also automatically recorded measurement data. For example, image data are recorded for a documentation of the status of a deceased person and for determining correct reasons for the death of the person. Advantageously, using the method according to the invention, a documentation of a pathology examination by a human expert can be supplemented by the result of an automatic processing algorithm.

A tumor-boarding offers an approach to treatment planning for malignancy in which a panel of physicians who are experts in various medical specialties, review and discuss a patient's medical condition and treatment options. The specialty of the participants of a tumor board depends on the respective disease. Regularly represented are oncologists, surgeons and other specialized physicians from different disciplines, for example surgery, dermatology, gynaecology, senology, urology, as well as radiologists, radiotherapists and pathologists. The resources for assessment and treatment planning may include user-generated electronic medical data, in particular free-text data and also automatically generated measurement based data, for example image data, which are used as basis for a finding. Such a finding itself can be used as basis for the assessment and treatment planning.

The result of the assessment and treatment planning is also referred to as an interdisciplinary definition of treatment strategies and subsequent feedback on the course of the disease not only benefit the patients, but also have an effect on further education and training for the doctors involved.

A therapy planning may include, additionally to user-generated electronic medical data, in particular free-text data, also automatically generated measurement based data, for example image data, which are used as basis for a finding. Such a finding itself can be used as basis for the therapy planning.

A patient monitoring includes technical measurement and monitoring systems for monitoring vital bodily functions of a patient. Further, also user-generated free-text based information can be used for documenting the condition of the patient. Hence, also a patient monitoring can be improved by concurrently generating, analysing and comparing the results of the analysis of user-generated and automatically generated medical data.

In case the specific medical process comprises a medical imaging process, the process preferably comprises at least one of:

- a radiological imaging,
- an oncological imaging,
- a dermatological imaging,
- an ophthalmological imaging.

Radiological imaging includes methods for imaging the inner parts of a human body. In addition to X-rays, other ionizing radiation, such as gamma rays or electrons, are also used. While an essential application is X-ray imaging, other imaging methods, such as sonography and magnetic resonance imaging, are also included in radiology, although no ionizing radiation is used in these methods.

Oncological imaging plays a central role in the diagnosis and therapy of many tumor diseases. This is an elementary component in the initial diagnosis of solid tumors and guides the image-guided biopsy for histological confirmation. In all other stages of the disease, non-invasive radiological diagnostics allow tumor follow-up and the assessment of a response to therapy. Oncological imaging includes an automatic generation of measurement data and also user-generated information of medical data are used for the examination based on the oncological imaging and therefore oncological imaging belong to the application field of the method according to the invention.

Dermatological imaging includes reflected light microscopy which has been established for many years for the diagnosis of skin tumors and pathogens firmly established because the higher larger details such as pigment distribution, blood vessels and even larger single cells are visible made. Sonography has evolved in the field proven in lymph node and vascular diagnostics, but high frequency ultrasound plays due to the insufficient resolution of dermal changes only a subordinate role. Dermatological imaging includes the automatic generation of measurement data. Also user-generated information of medical data are generated for an examination of these special type of imaging data and therefore they belong to the application field of the method according to the invention.

In addition to systemic cause clarification and therapy, ophthalmological diagnostics and imaging are of particular importance for the prognosis of an eye disease. In addition to routine ophthalmological examinations like visual acuity, slit lamp, fundoscopy, glaucoma diagnostics are important in clarifying the cause. In addition to traditional methods like tension, visual field, daily pressure profile, newer imaging methods of glaucoma diagnostics are also used. Comprehensive diagnostics today also include determining the central corneal thickness and, if appropriate, imaging the papilla using Heidelberg retina tomography or optical coherence tomography (abbreviated by OCT). OCT also plays an outstanding role in the follow-up and therapy monitoring of macular edema after venous retinal occlusion. The use of fluorescein angiography for venous occlusions is tried and tested, with e.g. the following information can be obtained: extent of the occlusion, degree of ischemia, presence and size of non-perfused areas, possible neovascularization formation and assessment of macular edema. All of the mentioned examinations comprising medical imaging can include the automatic generation of measurement data and also user-generated information of medical data and therefore belong to the application field of the method according to the invention.

In case the medical imaging process comprises a radiological imaging process, the radiological imaging comprises at least one of:

- a magnetic resonance imaging,
- a computer tomography,
- an OCT,
- a photoacoustic tomography,
- PET,
- SPECT,
- diffuse scattering tomography,
- infrared tomography,
- ultrawide bandwidth tomography,
- ultrasound.

Magnetic resonance imaging and computer tomography are well-known and widely used variants of radiological imaging and are therefore not described in detail at this point. OCT has been described above and is therefore not described again at this point.

Photoacoustic tomography is a biomedical imaging modality based on the photoacoustic effect. Non-ionizing laser pulses are delivered into biological tissues and part of the energy will be absorbed and converted into heat, leading to transient thermoelastic expansion and thus wideband ultrasonic emission.

PET is an acronym for positron emission tomography. PET is a functional imaging technique that uses radioactive substances known as radiotracers to visualize and measure changes in metabolic processes and in other physiological activities including blood flow, regional chemical composition and absorption.

SPECT is an acronym for single-photon emission computed tomography. SPECT is a nuclear medicine tomographic imaging technique using gamma rays. It is very similar to conventional nuclear medicine planar imaging using a gamma camera, but is able to provide true 3D information. This information is typically presented as cross-sectional slices through the patient.

Diffuse scattering tomography is a kind of diffuse optical imaging which is an imaging method using near-infrared spectroscopy or fluorescence-based methods. When used to create 3D volumetric models of the imaged material, diffuse optical imaging is referred to as diffuse optical tomography. The technique has many applications to neuroscience, sports medicine, wound monitoring and cancer detection.

Ultrawide bandwidth tomography includes optoacoustic imaging. Broadband optoacoustic waves generated by biological tissues excited with nanosecond laser pulses carry information corresponding to a wide range of geometrical scales. Typically, the frequency content present in the signals generated during optoacoustic imaging is much larger compared to the frequency band captured by common ultrasonic detectors, the latter typically acting as bandpass filters.

Ultrasound can also be used as separate means for generating medical images. An ultrasound image can be automatically analysed and in parallel analysed by an expert who generates user-generated electronic medical data.

Preferably, in the method for generating protocol data of a specific medical process, the user-generated electronic medical data comprise at least one of:

- text-based electronic medical data,
- electronic medical data generated by voice.
- audio-visually generated electronic medical data.

The above-mentioned forms of electronic medical data are typically generated for a medical report, preferably a radiological report. Such a medical report can comprise free text, it can also comprise a combination of marked image data, annotations and free text. For reducing the amount of work for a medical expert, the free text can also be generated by voice, for example using speech-to-text-software, or instead of free text, also audio data can be directly generated by voice. Advantageously, using the method according to the invention, these user-generated electronic medical data can be integrated in an automatic data processing and validation workflow without any restriction for the kind of generation of these data by the user. As mentioned above, also a mixture of audio data and video data, i.e. audio-visually generated electronic medical data, can be generated by a user. The visual electronic medical data may include a medical image and some marks and annotations of the user. The audio data include a comment of the user corresponding to the marked annotations or a comment replacing annotations of the user.

In a very preferred and very broadly applicable variant of the method according to the invention, the user-generated electronic medical data include a radiological report of a free text. Such a radiological report is generated for evaluating, preferably annotating medical image data, for example X-ray image data or magnetic resonance image data. Hence, the automatically created electronic medical data include a medical image on which the radiological report is based. In that variant, the automatic processing algorithm comprises an image processing algorithm which is applied to a medical image. Such an image processing algorithm can include an automatic extraction from the medical image or an automatic addition of information to the medical image. The image processing algorithm can include an automatic finding program which for example determines annotations referring to portions or locations of the electronic medical image data. Advantageously, the finding process of a user, in particular a radiologist, is technically supported by an automatic finding process, wherein the results of the radiologists and the automatic analysis process are brought in an integrated and further automatically processable form without additional work of a human person.

In a preferred variant of the method according to the invention the structured medical data objects comprise a graph structure. A graph is in graph theory an abstract structure that represents a set of items together with the connections existing between these items. A graph includes vertices as the mentioned items and edges as the connections or relationships between these items. Advantageously, such a logical structure enables to map most data structures used in medical data application, in particular the above-mentioned structured medical data objects.

Preferably, the graph structure comprises at least one of:

- a hierarchical graph structure,
- a cyclical graph structure,
- a medical knowledge graph structure, preferably a radiology knowledge graph structure.

A hierarchical graph structure includes a direction and a source, i.e. a vertex without incoming edges and a plurality of sinks, i.e. vertices without outgoing edges. Such a hierarchical graph for example includes a tree with directed edges. In particular, a DICOM SR object includes a hierarchical graph structure.

A cyclical graph structure includes a cycle, which is a nonempty trail in which only the first and last vertices are equal. In particular, a medical knowledge graph comprises a cyclical graph structure.

A medical knowledge graph structure comprises a cyclical graph structure and includes information about a plurality of different entities related to medical data and relationships between them. The different entities include medical questions, a plurality of different modalities for generating measurement data from a patient and a plurality of different medical analysis data, in particular radiographic findings. In particular, a special type of a medical knowledge graph, a radiology knowledge graph includes in a preferred variant at least three different classes classifying the different entities. Preferably, a first class includes different organs of a patient related to different medical questions, a second class includes different modalities for recording image data from the organs and a third class comprises different types of radiographic findings as different medical analysis data.

For implementing the method according to the invention using a medical knowledge graph structure, preferably, first, a global medical knowledge graph structure is generated. The global medical knowledge graph is not yet specified to a specified medical process. Next, a specific medical process, for example a medical imaging of a special region of the body of a patient, is determined. Based on the specific medical process, a subgraph structure is determined which is adapted to the current application.

The extraction and construction of a global medical knowledge graph can be implemented as follows:

first, facts from an extensive medical data source including a plurality of medical cases are extracted. The extensive medical data source can be understood as a kind of training corpus. Such an extensive medical data source preferably includes a medical reports database, preferably a radiology reports database, or a database for electronic health records of patient summaries. Facts include named entities such as disease findings, anatomy locations, severity scale and measurement data and relationships between the named entities. The entities become part of the vertices of the global medical knowledge graph. The relationships are represented by edges of the global medical knowledge graph. In particular for generating a radiology knowledge graph, facts from an extensive database including free text of a radiology report are extracted preferably using techniques for parsing the radiology report. Parsing techniques preferably include NLP techniques such as entity resolution and entity disambiguation.

Next, n-tuples, preferably triplets, are generated based on the extracted facts. N-tuples include the extracted entities and relationships between them. The generation of the n-tuples is implemented using methods of ontology learning. Ontology learning is the automatic or semi-automatic creation of ontologies. Ontology learning includes specific methods such as ontology extraction, formalization and refinement. Ontology extraction means that an ontology representing the training corpus is provided. Formalization and refinement are steps for providing additional constraints to the global medical knowledge graph. For example, contradictory or absurd entities or relationships are eliminated from the global medical knowledge graph. Formalization relies on domain specific knowledge available for e.g. through WebMD, or medical knowledge databases such as RadLex etc. WebMD is a kind of webbased lexicon pertaining to human health. RadLex includes a radiology lexicon comprising a comprehensive set of radiology terms for use in radiology reporting, decision support, data mining, data registries, education and research. Refinement is the process of checking for completeness and correctness of a global medical knowledge graph.

A further step of constructing the global medical knowledge graph is performed by adding new links, i.e. relationships, between the entities and determining corresponding confidences using statistical relational learning, which identifies missing links, i.e. relationships between the entities.

New links are generated as a part of learning from the training corpus. Confidence is the probability of a link between two entities. If two entities are strongly correlated, the confidence on the link is high.

Statistical Relational Learning is a collective of tasks such as link prediction, i.e. telling if two entities are related, entity clustering, i.e. categorizing entities based on relationships, and entity resolution, etc. Statistical Relational Learning is usually implemented through relational machine learning models such as Probabilistic Graph Models (abbreviated by the acronym PGM), Markov Logic Networks, Bayesian Neural Networks and Relational Dependency Networks.

The extraction of knowledge graphs from data sources and construction of knowledge graphs is described in Suchanek et al., 2013, "Advances in automated knowledge base construction", SIGMOD records journal, March and in Niklaus, C. et al., 2018, "A survey on open information extraction", arXiv preprint arXiv: 1806.05599.

Alternatively, one can employ also prior knowledge to construct static knowledge graphs such as illustrated in the work by Zhang et al., 2020, April, "When radiology report generation meets knowledge graph", in Proceedings of the AAAI Conference on Artificial Intelligence, vol. 34, No. 07, pp. 12910-12917.

One can also use a combination of prior knowledge with auxiliary information from a related ontology databased such as MeSH as shown in Hou et al., 2021, "Multi-Label Learning with Visual-Semantic Embedded Knowledge Graph for Diagnosis of Radiology Imaging", IEEE Access, 9, pp. 15720-15730.

Alternatively, one can employ generic information extraction and reasoning tools such as Open IE, DeepDive or Interactive Knowledge Extraction. Generic information extraction is referring to information extraction methods that are generalized and not domain specific.

In natural language processing, Open IE, i.e. open information extraction, is a task of generating a structured, machine-readable representation of the information in text, usually in the form of triples or n-tuple propositions. Open IE is described in Banko, Michele et al, (2007), "Open Information Extraction from the Web", Conference on Artificial Intelligence.

DeepDive is a system to extract value from dark data. Dark data is the great mass of data buried in text, tables, figures and images, which lacks structure and so is essentially unprocessable by existing conventional software. DeepDive helps to bring dark data to light by creating structured data from unstructured information and integrating such data with an existing structured database. DeepDive is described in http://deepdive.stanford.edu/.

Interactive Knowledge Extraction, abbreviated as IKE is described in Dalvi, B. et al, 2016, June, "IKE- an interactive tool for knowledge extraction", in proceedings of the 5th Workshop on Automated Knowledge Base Construction (pp. 12-17).

Preferably, the concept of a medical knowledge graph is applied to the use of a radiology knowledge graph which is related to medical imaging of the inside of a body of a patient.

The construction of a first and a second radiology knowledge graph as a particular type of the first and the second structured medical data object is preferably performed based on a generic structure named global radiology knowledge graph. The generation of the global radiology knowledge graph is performed based on a generalized database related to a plurality of cases and patients.

For construction of a global radiology knowledge graph, the above-mentioned more generic steps for constructing a medical knowledge graph are described in more detail.

The step of extracting facts includes the collection of a large training corpus of radiology reports and parsing the radiology reports into relevant sections.

A radiology report usually has multiple subtopics related to different sections that are reported by the radiologist such as description of patient's clinical condition or the technical quality of the examination. Parsing the radiology report is the process of extracting sub-text that is associated with these different sections. For the case of a radiology report a relevant section can comprise: patient demographics, clinical history, comparison to prior exams, technique used, examination quality, differential diagnosis, conclusions, and recommendations. The membership to these sections is used to describe attributes of named entities in the extracted global radiology knowledge graph.

Certain named entities could occur in multiple sections. So associating which section the named entity came from is important while comparing one radiology knowledge graph with another radiology knowledge graph. Attributes of named entities could include description of the named entity. For e.g. a lung nodule is a named entity and its attributes could be diameter, margin, spiculation shape etc.

The steps of formulating n-tuples includes applying methods for knowledge graph extraction from structured or unstructured text using machine learning methods such as probabilistic graphic models, random walker methods such as Path Ranking Algorithm, ProPPR (which is an acronym for Programming with Personalized PageRank), embedding techniques such as matrix factorization tensor factorization and compositional neural models. This substep is used for establishing relationships between the entities in the global knowledge graph.

In particular for the generation of a global radiology knowledge graph, the step of formulating n-tuples may also include the provision of a plurality of different sets of image data as a kind of training data that are used as basis for the construction of the global radiology knowledge graph. The construction includes the step of parsing image metadata for attributes such as modality, body part and protocol, to associate them as global entities.

Meta data include data that provide information about other data, but not the content of the data, such as the text of a radiology report or the image data itself. In the above-mentioned context, an image illustrating an organ may also include additionally to the image itself information about the modality used for imaging the imaged body part and the protocol used for controlling the medical imaging.

The method of Path Ranking Algorithm is described in Lao et al, 2011, July, "Random walk inference and leaning in a large scale knowledge base", in proceedings of the 2011 conference on empirical methods in natural language processing (pp. 529-539) .

The method of ProPPR is described in Wang et al., 2013, October, "Programming with personalized pagerank: a locally groundable first-order probabilistic logic", in proceedings of the 22nd ACM international conference on Information & Knowledge Management (pp. 2129-2138).

The step of formulating n-tuples may further include a refinement of an extracted global radiology knowledge graph using ontological knowledge of the domain, i.e. the special field, as encoded in an ontology schema such as MeSH, RadLex, UMLS.

For refinement, probabilistic graphic models are described in Puja et al., 2013, "Large-Scale Knowledge Graph Identification Using PSL", in AAAI Fall Symposia and in Jiang, S. et al, 2012, December, "Learning to refine an automatically extracted knowledge base using Markov logic", in 2012, IEEE, 12th International Conference on Data Mining (pp. 912-917).

UMLS is an acronym for Unified Medical Language System. UMLS is a compendium of many controlled vocabularies in the biomedical sciences. It provides a mapping structure among these vocabularies and thus allows one to translate among the various terminology systems. It has the function of a comprehensive thesaurus and ontology of biomedical concepts. UMLS further provides facilities for natural language processing. UMLS consists of knowledge sources and a set of software tools.

UMLs is described in Lindberg, C., 1990. The Unified Medical Language System (UMLS) of the National Library of Medicine. Journal (American Medical Record Association), 61(5), pp.40-42.

Further, also the step of constructing a global medical knowledge graph with new links, i.e. relationships and confidences may include a refinement of the radiology knowledge graph using ontological knowledge of the domain as encoded in ontology schema such as MeSH, RadLex, UMLS. An ontology scheme such as RadLex not just introduces entities such as radiological findings, body part etc., but also has domain knowledge creating relationships between them.

Based on the process described above, a generic, i.e. a global radiology knowledge graph is constructed. For the next step, it is assumed that an appropriate radiology knowledge graph is learnt. An appropriate radiology knowledge graph is a radiology knowledge graph constructed for the task at hand. For example, for comparing radiology reports, we assume that the task of learning the radiology knowledge graph is done using the correct medical training corpus using the steps defined. Also, for radiology reports, one should not use a pathology knowledge graph.

Such a global radiology knowledge graph can be used as generic template for an automatic peer review of a radiology report.

Next, the current case of use starts with receiving medical image data of a patient acquired from a modality. The medical image data are usually combined with corresponding image meta data. The image meta data comprise data related to medical image data. For example, image meta data can be received via DICOM communication.

The next step includes the generation of an appropriately selected subgraph structure based on the received image meta data. Since the exploration of a global radiology knowledge graph is difficult due to its large size and due to a lack of clinical relevance of a number of vertices to a current use case, a reduction of the size of the global radiology knowledge graph to a relevant subgraph is performed. For achieving the subgraph, the received image meta data are used to get information about attributes of relevant entities of the subgraph. In this way, relevant types of entities of the subgraph can be inferred and the remaining types of entities of the global radiology knowledge graph which are not inferred by the image meta data and seem to be therefore not relevant for the received medical image data are left out. Hence, a subgraph with a reduced graph structure compared to the global radiology knowledge graph is generated. The subgraph can be used as basis for a first subgraph related to the user-generated electronic medical data, in particular the radiology report and a second subgraph related to the automatically processed medical image data.

For generating the first subgraph, the radiology report is also examined to extract relationships that have been reported as present or absent or not-reported. Also attributed entities can be taken from the radiology report. The extraction of relationships and attributed entities can be realized by tokenization approaches to identify key parts of speech, named entities such as anatomy, radiographic findings and differential diagnosis. Tokenizing, dependency parsing and mapping to the first subgraph by matching detected named entities and relationships, i.e. "present", "absent", "not-reported" or "uncertain", into edge weights can be accomplished by using tools such as Stanford CoreNLP. Stanford CorNLP is described in Manning, C.D. et al., 2014, June, "The Stanford CoreNLP natural language processing toolkit", in Proceedings of 52nd annual meeting of the association for computational linguistics: system demonstrations, (pp. 55-60) .

For generating the second subgraph, i.e. a knowledge graph generated by applying an automatic processing algorithm to the received medical image data, relationships between the nodes, i.e. vertices, are linked and weighted based on AI- or ML-based automated computer aided detection tools (ML is an acronym for machine learning). To this end, a number of key features are extracted using computer aided detection or diagnostic tools as edge weights, i.e. weights of relationships. The edges are connected based on different rules. Such a rule can include the order that if radiographic findings co-occur in the same anatomy, then the related nodes are linked with a bidirectional connection. Alternatively, relationship can also be established based on conditional probabilities estimated using AI-based methods for computer aided detection and diagnosis of particular radiographic findings in body regions.

Based on the first subgraph and the second subgraph, a comparison, in particular a graph matching is performed to find all the elements that differ significantly between the first and second subgraph. It can be started by randomly parsing the second subgraph into smaller sub-subgraphs and trying to find a match to the first subgraph, preferably using the Hungarian algorithm or variants of it. This algorithm is described in https://en.wikipedia.org/wiki/hungarian-algorithm. The matching can also be accomplished using methods such as SimRank, similarity flooding, etc. If a match is found, then this is deemed as information already reported. If a match is not found, a deviation between the first and the second subgraph is detected and the deviation is added to a third subgraph, named difference graph. After a certain number of iterations, the difference graph tends toward isomorphism. Isomorphism means in that context that after some iterations there is no additional difference found, i.e. the difference graph does not change any more.

Preferably, the generated difference graph is converted into human readable clinical text, in particular a text of a radiology report, using a rule based method using templates with named entities as placeholders. An alternative to rule based methods is to employ deep learning models, for example using a graph convolution encoder feeding to a text generation recurrent neural network-based decoder.

After training the AI-based operations for generating the first subgraph and the second subgraph, for generating the difference graph, the method according to the invention can be deployed as a micro-service that is triggered in the backend as soon as a particular case is sent to a clinical workflow list, preferably a radiology workflow list.

Once a case has been reported, a difference report based on automatically generated, preferably AI-based peer reviewed data can be shown to the user who generated the radiology report. Preferably, the difference report is either shown on a viewer, in particular a PACS/RIS window (RIS is an acronym for Radiology Information System), or as a pop-up message or before case-closeout as a confirmatory step. In a fully automated workflow, the automatically generated peer review report can be appended to the original radiology report, as a second opinion beyond the user-generated medical data generated by the user.

It has to be mentioned that the described variant can not only be employed to a radiology report, but also be employed to other user-generated text-based medical data assigned to medical image data.

Structured medical data objects broadly used in medical data application preferably comprise a predefined structure.

Those predefined structured medical data objects include the following types:

- a DICOM SR object,
- an HL7 object,
- an FHIRcast object,
- an XML object.

A DICOM SR object is broadly used for the communication and management of medical imaging information and related data. DICOM is most commonly used for storing and transmitting medical images enabling the integration of medical imaging devices such as scanners, servers, workstations, printers, network hardware and image archiving and communications systems like PACS from multiple manufacturers. A DICOM SR object can be represented by a hierarchical graph structured as mentioned-above. Advantageously, since the structure of a DICOM SR object is predefined, the generation of a DICOM SR object based on automatically processed automatically created electronic medical data, in particular image data, can be easily performed using a particular template such as CAD-SR or Measurements Report TID1500.

The method according to the invention can also be applied to non-image attributes, e.g. values found in textual medical data like HL7 messages or FHIR resources concerning the order or the observations, for example order comments, procedure codes and descriptions, etc.

In HL7 these could include an OBR.13 segment containing clinical information about a patient or specimen, OBR.18 and OBR.19 segments concerned to a custom placer field and OBR.20 and OBR.21 concerned to a custom filler field in diagnostic service or an OMI_O23 Imaging order related to all NTE segments concerning notes and comments about the patient, the order, etc. This order is used in communication between the information systems involved in the fulfillment of a request directed to an imaging department, such as a Radiology Information System (RIS) and a Picture Archiving and Communication System (PACS).

HL7 (an acronym for Health Level Seven) refers to a set of international standards for transfer of clinical and administrative data between software applications used by various healthcare providers. These standards focus on the application layer which is "layer 7" in the OSI model. The HL7 standards are produced by Health Level Seven International, an international standards organization and are adopted by other standards issuing bodies such as American National Standards Institute and International Organization for Standardization.

FHIR is an acronym for "Fast healthcare Interoperability Resources" and includes a standard developed based on HL7. It describes formats and elements and an application programming interface for exchanging electronic health records. FHIRcast describes an application which synchronizes healthcare applications in real time to show the same clinical context to a common user.

XML is an abbreviation of "Extensible Markup Language". XML is a markup language and file format for storing, transmitting and reconstructing arbitrary data. It defines a set of rules for encoding documents in a format that is both humanreadable and machine-readable.

If the user-generated electronic medical data comprise text-based electronic medical data and the structured medical data objects comprise a well-known predefined structure, a complex construction of a global structure for the structured medical data objects is not necessary. In that case the step of generating a first structured medical data object is preferably implemented by content extraction, comprising the steps of:

- parsing the text-based electronic medical data and
- generating the first structured medical data object based on the parsed text-based electronic medical data, wherein the parsed text-based electronic medical data are grouped into structures which form the first structured medical data object.

To bridge the gap between automatically, preferably AI-generated structured medical data objects vs. human text-based reporting, the proposed invention utilizes parsing of text-based electronic medical data, preferably parsing a free text DICOM report, i.e. a special kind of radiological report.

Preferably, Natural Language Processing (with the corresponding acronym NLP) is used to parse the text-based electronic medical data, in particular a free text DICOM report, to map it to standardized semantic concepts, in particular concepts used for DICOM SR, encoding along with the right encoding concept items. Upon successful parsing, preferably, an encoding tree is constructed to map to a predefined standardized medical data object structure, in particular a DICOM SR object structure, with concept items and relationships between them. The structured medical data object, in particular a DICOM SR object, generated by parsing from the text-based electronic medical data, in particular a radiological report and preferably a free text DICOM report, is then compared and contrasted with the automatically, preferably AI-generated structured medical data object, in particular a DICOM SR object. If there is a discrepancy found, the measurement of the automatic processing algorithm needs to be reconciled with human measurement. The structured medical data objects are then compared or merged to form a final human verified structured medical data object, in particular a DICOM SR object, that can then be optionally committed to an electronic health record of the patient. This workflow is fully automated and may happen in the background as a particular case is being reported. Hence, it does not create additional burden to the radiologist.

In detail, the parsing step preferably includes at least one of:

- clinical word-sense disambiguation for acronyms and abbreviations in the text-based electronic medical data,
- semantic mapping of semantically similar terms in the text-based electronic medical data to standardized terms,
- optionally machine translation of the text-based electronic medical data,
- clinical named entity recognition to locate and classify clinical named entities mentioned in the text-based electronic medical data into pre-defined categories.

Different steps of processing text-data, i.e. word-sense disambiguation, semantic mapping and clinical named entity recognition are described in detail in the following scripts:

Wu, S., Roberts, K., Datta, S., Du, J., Ji, Z., Si, Y., Soni, S., Wang, Q., Wei, Q., Xiang, Y. and Zhao, B., 2020. Deep learning in clinical natural language processing: a methodical review. Journal of the American Medical Informatics Association, 27(3), pp.457-470 and

Wang, Y., Wang, L., Rastegar-Mojarad, M., Moon, S., Shen, F., Afzal, N., Liu, S., Zeng, Y., Mehrabi, S., Sohn, S. and Liu, H., 2018. Clinical information extraction applications: a literature review. Journal of biomedical informatics, 77, pp.34-49.

Clinical anatomical entities include the following types of clinical named entities: clinical finding, imaging modality, imaging observation, procedure and non-anatomical entities.

In a variant of the method according to the invention the step of creating the first structured medical data object includes at least one of:

- mapping recognized clinical entities to concept items, i.e. name/value pairs,
- extracting relationships from the parsed data between each concept item, i.e. name/value pair,
- constructing the first structured medical data object based on the concept items and the extracted relationships.

Concept items including name/value pairs are determined using dictionary or rule-based methods, for example clinical linguistic-grammar methods. Typical concept items include name/value pairs, comprising text, numerics, code, image and spatial coordinates. Advantageously, semantic information can be extracted from the text-based electronic medical data for generating the first structured medical data object.

As mentioned above, an important variant of the method according to the invention realizes a "peer review" of user-generated medical electronic medical data, preferably a radiological report. In that variant, the step of performing actions preferably includes generating a combined structured medical data object indicating deviations between the first and second structured medical data objects. The deviations may include contradictory concept items and/or relationships of the first and second structured medical object. Advantageously, the result of "peer review" is transferred into a structure which can be easily automatically processed further.

Based on the combined structured medical data object, text-based electronic medical data, i.e. a peer-reviewed text-based data, preferably a peer reviewed radiological report, are generated. Advantageously, the result is generated in an easily readable form.

Further, the user, for example an expert, is provided with the peer-reviewed text-based electronic medical data, preferably the peer-reviewed radiological report. Hence, the user is enabled to correct his own text-based electronic medical data, preferably a radiological report, without any effort of another colleague and without the risk of a personal conflict between the user and a peer reviewing colleague.

Alternatively, the actions comprise the step of archiving at least one of the automatically generated protocol data. In case the protocol data include a third structured medical data object, the third structured medical data object is preferably archived. Preferably, the third structured medical data object includes a DICOM SR object and the DICOM SR object is archived in an electronic health record of the patient. In case the protocol data include text-based electronic medical data, these text-based electronic medical data are transformed in a standardized data form. Advantageously, the protocol data are archived in a mostly compatible manner, such that a typical clinical data managing system is able to further process these generated data.

In case the first structured medical data object is generated based on text-based electronic medical data, the automatically peer-reviewed first structured medical data object may be archived. Alternatively or additionally, the peer-reviewed text-based electronic medical data are archived.

In a variant of the method according to the invention the step of generating the protocol data includes a step of merging the first structured medical data object and the second structured medical data object.

In a particular realization of such a merging approach, in particular a graph linking approach, the following steps are performed: Given a first structured medical data object based on user-generated electronic medical data, in particular a first graph $G_1 = (V_1, E_1, X_1)$, and a second structured medical data object generated by applying an automatic processing algorithm to automatically created electronic medical data, in particular a second graph $G_2 = (V_2, E_2, X_2)$, we intend to construct a composite structured medical data object, in particular a third graph $G_\emptyset$, that is merged from the two input structured medical data objects, in particular the first graph $G_1$ and the sec-

ond graph $G_2$. The vertices of the composite third graph $G_\emptyset$, are a simple union of the incoming vertices $V_\emptyset = V_1 \cup V_2$, i.e. the sets of vertices of the first and second graph $G_1$, $G_2$. In other words, a topologically sorting of the objects, in particular the graphs $G_1$ and $G_2$, is performed. $X_1$, $X_2$ are sets of features of vertices. $V_1$, $V_2$ are sets of vertices and $E_1$, $E_2$ are sets of edges, i.e. relationships.

If vertices are connected in any of the two incoming graphs by the sets of edges $E_1$, $E_2$, a resulting set of edges $E_\emptyset$ is established.

For the set of features $X_\emptyset$ at the vertices, say $x_i$

$$\in X_\emptyset, x_i = f\left(x_i^1, x_i^2, N(v_i^1),\ N(v_i^2)\right)$$ , wherein $N$

is a connected neighborhood of the incoming graphs and

$x_i^1 \in X_1$, $x_i^2 \in X_2$, $v_i^1 \in \mathbb{V}_1$ and $v_i^2 \in \mathbb{V}_2$ .

Neighborhood is defined around a vertex. k-order neighborhood of a vertex is defined as all the vertices that are connected exactly by k edges from the vertex. For k = 1, then we only consider information from the immediate neighbors of the vertex. For k=2, we consider information from immediate neighbor vertices and also the immediate neighbor vertices of the neighbors of the vertex.

The function $f$ can be realized with multiple approaches:
Using heuristics, an object merge and edit, in particular a graph merge and edit, can be rule based. Such a rule can provide that user-generated results always supersede automatically generated results in times of conflict. If a conflict exists, automatically generated items are overwritten by user-generated concept items.

Further, a merge can be achieved using graph editing methods such as Bellman-Ford and Dijkstra's algorithm.

Furthermore, a merge can be implemented using machine learning methods such as hierarchical clustering or graph neural networks.

Analogue to a preferred variant of the method according to the invention, preferably, the protocol data generation device according to the invention comprises a deviation detection reaction unit for performing a deviation reaction based on the generated protocol data generated based on detected deviations between the first structured medical data object and the second structured medical data object.

Advantageously, technique-based actions can be performed based on the result of the generated protocol data and detected deviations. Hence, the deviation detection reaction unit preferably includes technical means for implementing a deviation detection reaction, if deviations are determined.

Such a technical means for deviation detection reaction includes preferably, at least one of the following means:

- an alert unit for generating an alert output for a user,
- an amendment unit for generating amended protocol data,
- a marking unit for

  - generating protocol data including marks indicating deviations, and/or for
  - including measurement data with marked deviations into the protocol data.

The invention is explained below with reference to the figures enclosed once again. The same components are provided with identical reference numbers in the various figures.

The figures are usually not to scale.

FIG 1 shows a schematic view including symbols illustrating a method for generating protocol data of a specific medical process according to an embodiment of the invention,

FIG 2 shows a schematic view illustrating the step of generating a first structured medical data object,

FIG 3 shows a flow chart diagram illustrating details of the step of generating a first structured medical data object of the method for generating protocol data of a specific medical process according to the invention,

FIG 4 shows a schematic view illustrating the step of generating a second structured medical data object of the method for generating protocol data of a specific medical process according to the invention,

FIG 5, which is divided in FIG 5A and FIG 5B, shows a schematic view illustrating the step of generating a merged structured medical data object of the method for generating protocol data of a specific medical process according to the invention,

FIG 6 shows a schematic view on a protocol data generation device according to an embodiment of the invention,

FIG 7 shows a schematic view including symbols illustrating the method for generating protocol data of a specific medical process according to an embodiment of the invention, wherein peer reviewed radiological report data are generated,

FIG 8 shows a schematic view on a global radiology knowledge graph, representing the content of a radiological report,

FIG 9 shows a schematic view illustrating a reduction of a global radiology knowledge graph to two different subgraphs,

FIG 10 shows a flow chart diagram illustrating the method for generating protocol data of a specific medical process according to an embodiment of the invention, wherein a peer reviewed protocol is generated,

FIG 11 shows a schematic view on a medical process system according to an embodiment of the invention.

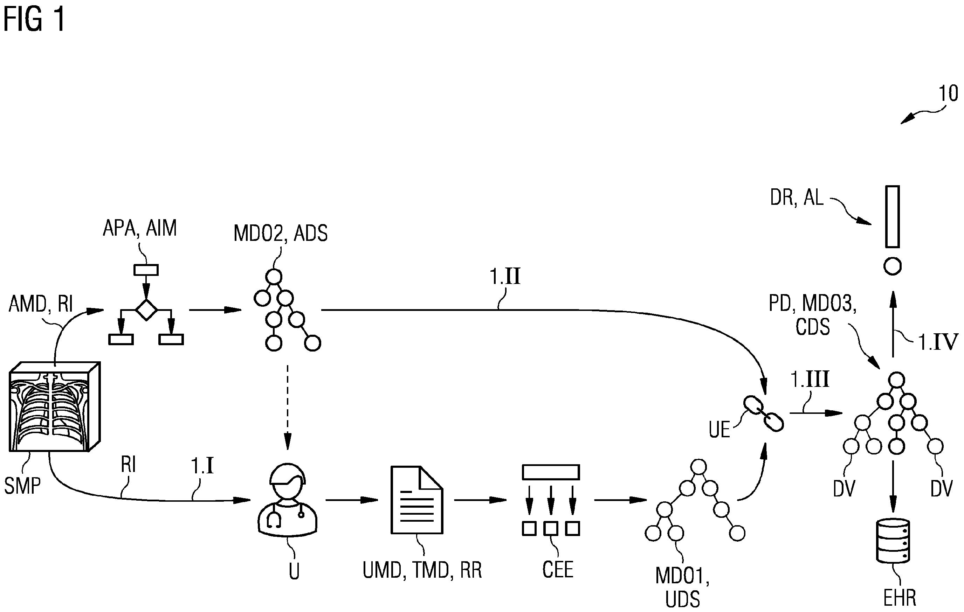

In FIG 1, a scenario 10 illustrating the method for generating protocol data PD of a specific medical process SMP in an embodiment using DICOM SR objects ADS, UDS, CDS as special type of structured medical data objects MDO1, MDO2, MDO3 is illustrated. The method includes two different lines of action. A first line for generating a DICOM SR object UDS based on user-generated data represented by step 1.I and a second line for generating an AI-generated DICOM SR object ADS represented by step 1.II. At the connection point of the different lines of action the different DICOM SR objects UDS, ADS are merged to a composite result, i.e. to a final composite DICOM SR object CDS.

In FIG 1, the lower part is related to a semi-automatic generation of a DICOM SR object UDS based on user-generated data representing step 1.1 of the method according to the invention and the upper part is assigned to step 1.II, wherein a model based fully automatic generation of an AI-generated DICOM SR object ADS is performed. The common source for the different lines of action 1.1, 1.II is a radiology image RI of a patient. A radiology image RI represents a documentation of a specific medical process SMP, in this case a medical imaging process.

In the lower line of action, representing step 1.1 of the method according to the invention, the radiology image RI is analysed by a user U, in this special embodiment a radiologist. The user U usually dictates or transcribes an impression of the radiology image RI and in particular a description of annotations or segmentations and findings and a summary thereof on a PACS (PACS is an acronym for Picture Archiving and Communication System) using a dictation tool and generates a free-text radiology report RR in this manner. The free-text radiology report RR is a special type of text-based electronic medical data TMD, which is a special type of user-generated medical data UMD.

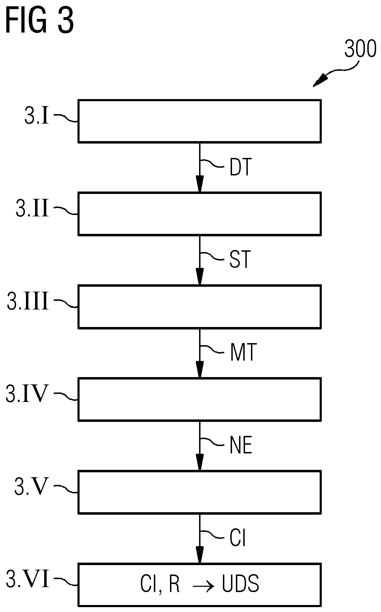

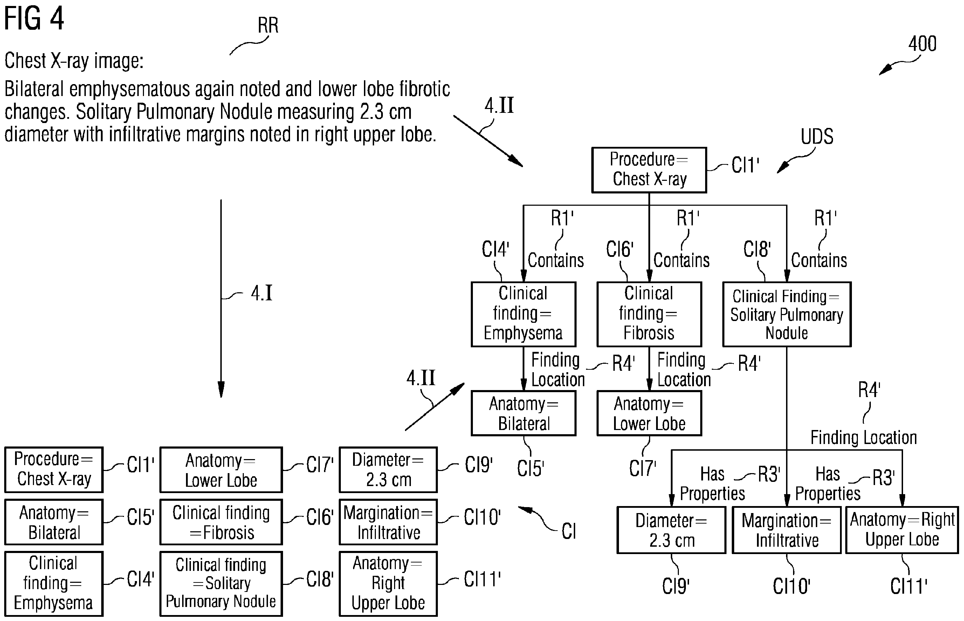

For transferring that informal information of a free-text radiology report RR into a structured formalized data structure, a concept extraction engine CEE is used. Such a concept extraction engine CEE automatically extracts formalizable information, i.e. concept items and relationships, which are later discussed in detail, out of the free-text radiology report RR by parsing the free-text radiology report RR. The substep of parsing can be implemented using Natural Language Processing on the free-text radiology report RR. Then, the parsed information is assigned to the structure of a structured medical data object MDO1, in particular a DICOM SR object UDS generated based on user-generated data. Detailed steps of the concept extraction are described in context with FIG 3 and FIG 4.

The radiology image RI, which is a particular type of automatically created electronic medical data AMD, is also processed based on an AI-model AIM. The AI-model AIM defines the rules of an automatic processing algorithm APA. The automatic processing algorithm APA is used to automatically generate a second structured medical data object MDO2, in particular an AI-generated DICOM SR object ADS by automatically processing image data of the radiology image RI as shown in the upper part of FIG 1. In particular, the automatic processing algorithm APA includes an annotation and finding based on the radiology image RI.

As one can further take from FIG 1, the AI-generated DICOM SR object ADS and the DICOM SR object UDS generated based on user-generated data are then merged using an SR update engine UE in step 1.III. The SR updating includes also an editing of conflicts between the AI-generated DICOM SR object ADS and the DICOM SR object UDS based on user-generated data. Hence, a final composite DICOM SR object CDS is generated based on the AI-generated DICOM SR object ADS and the DICOM SR object UDS generated based on user-generated data. The final composite DICOM SR object CDS is a special type of a third structured medical data object MDO3, which is in turn a special type of protocol data PD. If deviations DV between the AI-generated DICOM SR object ADS and the DICOM SR object UDS generated based on user-generated data exist, these deviations are received and marked in the final composite DICOM SR object CDS.

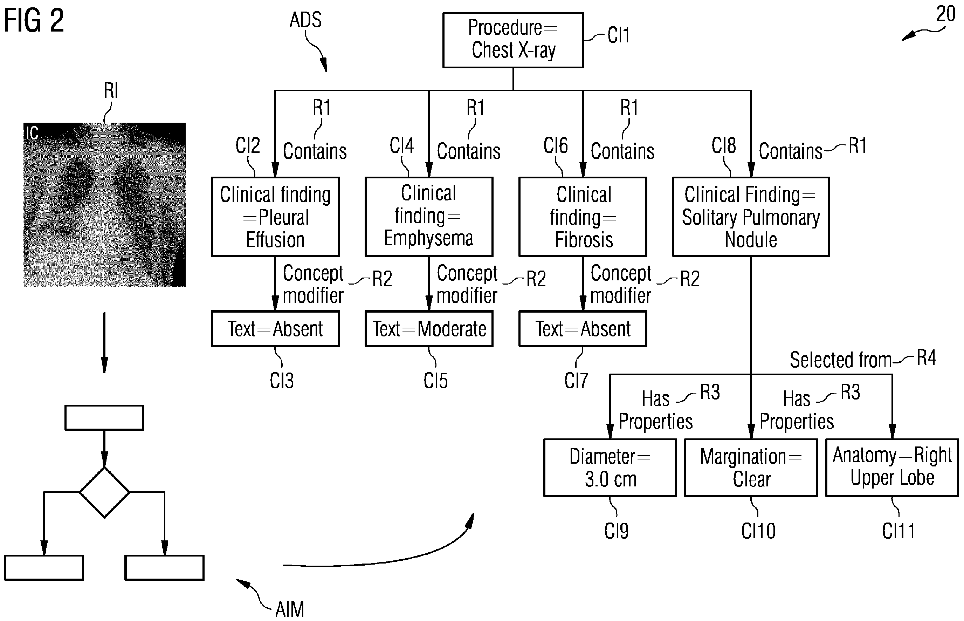

The DICOM SR objects ADS, UDS, CDS can be represented as directed acyclic graph objects with vertices including concept items and edges representing the relationships between the concept items as later illustrated in detail in FIG 2, FIG 4 and FIG 5. The final composited graph or DICOM SR object CDS can be realized such that data of the DICOM SR object UDS generated based on user-generated data always supersedes data of the AI-generated DICOM SR object ADS in times of conflict. If a conflict exists, AI-generated concept items are overwritten by user-generated DICOM SR concept items. If there is no conflict, the graph vertices including the concept items and the edges including relationships between the concept items of the different AI-generated DICOM SR object ADS and the DICOM SR object UDS generated based on user-generated data are merged. As already mentioned, for the merging process, also more advanced graph linking approaches can be implemented such as hierarchical clustering, graph neural networks etc. Optionally, the generated final composite DICOM SR object CDS is archived in an electronic health record EHR of the patient for archival purposes.

In step 1.IV, an alert AL for the user U is generated as a special kind of deviation detection reaction DR based on the result of step 1.III. For example, deviations DV are also displayed to the user U such that the user U can decide if it is reasonable to amend the user-generated DICOM SR object UDS and in particular the radiology report RR.

In FIG 2, the upper line of action of FIG 1 assigned to step 1.II, comprising the generation of an AI-generated DICOM SR object ADS using an AI-model AIM for processing a radiology image RI, is illustrated in an enlarged view 20. The AI generated DICOM SR object ADS comprises nodes or vertices including concept items CI1, CI2, ..., CI11 and edges including relationships R1, R2, R3, R4 between neighboured vertices. The AI-generated results are organized in a hierarchical fashion of concept items based on data types as for example coded nomenclature, numeric measurements, spatial and temporal ROI references, automated analysis results, procedure log, radiation report and based on data analysis techniques as for example key object selection etc.

The concept items CI1, CI2, ..., CI11 are illustrated in an explicit way in the following table:

CI1 Procedure = Chest X-ray
CI2 Clinical finding = Pleural Effusion
CI3 Text = absent
CI4 Clinical finding = Emphysema
CI5 Text = Moderate
CI6 Clinical finding = Fibrosis
CI7 Text = Absent
CI8 Clinical Finding = Solitary Pulmonary Nodule
CI9 Diameter = 3.0 cm
CI10 Margination = Clear
CI11 Anatomy = Right Upper Lobe.

The relationships R1, R2, R3, R4 are illustrated in an explicit way in the following table:

R1 contains
R2 concept modifier
R3 has properties
R4 finding location.

Hence, the AI-generated DICOM SR object ADS includes the following chains of information:

CI1, R1, CI2, R2, CI3: Procedure = Chest x-ray, contains Clinical finding = Pleural Effusion, concept modifier Text = Absent.

CI1, R1, CI4, R2, CI5: Procedure = Chest x-ray, contains Clinical finding = Emphysema, concept modifier Text = Moderate.

CI1, R1, CI6, R2, CI7: Procedure = Chest x-ray, contains Clinical finding = Fibrosis, concept modifier Text = Absent.

CI1, R1, CI8, R3, CI9, R3, CI10, R4, CI11: Procedure = Chest x-ray, contains Clinical Finding = Solitary Pulmonary Nodule, has properties Diameters = 3.0 cm, has properties Margination = Clear, selected from Anatomy = Right Upper Lobe.

In FIG 3, a flowchart diagram 300 is shown, illustrating detailed steps of concept extraction from text-based medical data TMD, in particular a radiology report RR. Concept extraction is used in step 1.1 shown in FIG 1.

In step 3.1, a clinical word-sense disambiguation for acronyms and abbreviations based on the text of the radiology report RR is performed using natural language processing techniques for generating disambiguated terms DT.

For example, in the text of the radiology report RR attributes in Chest X-ray images, the synonymous terms such as "PA", "P.A.", "pa", "post. Ant." etc., are disambiguated to a standardized term "Posterior Anterior" that can be defined for example from a list of pre-specified terms.

In step 3.II, a semantic mapping to standardized terms ST is performed. In this step, semantically similar terms are mapped to a standardized terminology to transform the similar terms into a computer readable and searchable identifier. The most semantically closest standardized term is determined using available ontologies or privately defined dictionaries. Examples of useful tools are a privately curated ontology, for example, a private list of body parts or a standard ontology like RadLex, SNOMED-CT, LOINC or UMLS.

The acronym SNOMED-CT stands for Systematized Nomenclature of Medicine Clinical Terms and is currently one of the most comprehensive and important medical terminologies worldwide.

The acronym LOINC stands for Logical Observation Identifiers Names and Codes and represents a database and a universal standard for identifying medical laboratory observations. LOINC applies universal code names and identifiers to medical terminology related to electronic health records. The purpose is to assist in the electronic exchange and gathering of clinical results such as laboratory tests, clinical observations, outcomes management and research.

In step 3.III, a machine translation MT of the text of the radiology report is performed. If the reporting language is not English, the incoming disambiguated and semantically mapped text of the radiology report RR needs to be translated into English language. For that purpose, neural machine translation tools and techniques typically built using sequence to sequence models can be used.

In step 3.IV, clinical named entity recognition is performed. Step 3.IV is performed to locate and classify named entities NE mentioned in unstructured free text into pre-defined categories, also named concepts, such as anatomical entity, clinical finding, imaging modality, imaging observation, procedure, non-anatomical entities etc. For example, using the named entity recognition for analysing a disambiguated free-text report, in particular a disambiguated imaging procedure description of chest x-ray images, named entity recognition groups and categorizes related words such that words assigned to the same group or category can be represented by the same concept item in the next step.

In step 3.V, a mapping to concept items CI, i.e. pairs of a concept and a value, is performed. After identifying clinical entities and mapping them to standard lexicons in step 3.IV, in step 3.V, concept items CI for DICOM SR objects are generated from the radiology report RR. This is done using dictionary or rule-based methods or using clinical linguisticgrammar methods. The concept items CI in a DICOM SR object usually comprise text, numerics, code, image spatial coordinates etc. These concept items CI are shown in FIG 4. The concept items CI are the vertices or nodes of a hierarchical acyclic graph, in particular representing a DICOM SR object UDS.

In step 3.VI, relationships R between each concept item CI are established to construct a hierarchical tree structure as supported by the standard of DICOM SR. For each of the entities, DICOM SR supports relationships R such as: "contains", "has observation context", "has concept modifier", "has properties", "has acquisition context", "inferred from" and "selected from" and "finding location". The method for extracting such a relationship in free text can be realized based on rule-based relationship extraction, for example implemented by parts-of-speech-tagging, word sequence patterns or by learning based methods including weakly supervised, supervised, distantly supervised or unsupervised approaches.

Rule-based relationship extraction is described in the following documents:

Waltl, B., Bonczek, G. and Matthes, F., 2018. Rule-based information extraction: Advantages, limitations, and perspectives. Jusletter IT (02 2018) and

Chiticariu, L., Li, Y. and Reiss, F., 2013, October. Rule-based information extraction is dead! long live rule-based information extraction systems!. In Proceedings of the 2013 conference on empirical methods in natural language processing (pp. 827-832).

FIG 4 shows an illustration 400 of a full pipeline of transforming an incoming free-text radiology report RR into a DICOM SR object UDS generated based on user-generated data. FIG 4 can be understood as a detailed description of the second part of step 1.1 in FIG 1. In step 4.1, the free-text radiology report RR is parsed to extract concept items, i.e. coded name value pairs CI1', CI4', ..., CI11' as described in context with FIG 3. In step 4.II, relationships R1', R3', R4' between the concept items CI1', CI4', ..., CI11' are determined. The final representation of the DICOM SR object UDS is arranged in form of a hierarchical tree with the imaging procedure as the root vertex represented by the first concept item CI1'.

The concept items CI1', CI4', ..., CI11' are illustrated in an explicit way in the following table:

CI1' Procedure = Chest X-ray
CI4' Clinical finding = Emphysema
CI5' Anatomy = Bilateral
CI6' Clinical finding = Fibrosis
CI7' Anatomy = Lower Lobe
CI8' Clinical Finding = Solitary Pulmonary Nodule
CI9' Diameter = 2.3 cm
CI10' Margination = Infiltrative

CI11' Anatomy = Right Upper Lobe.

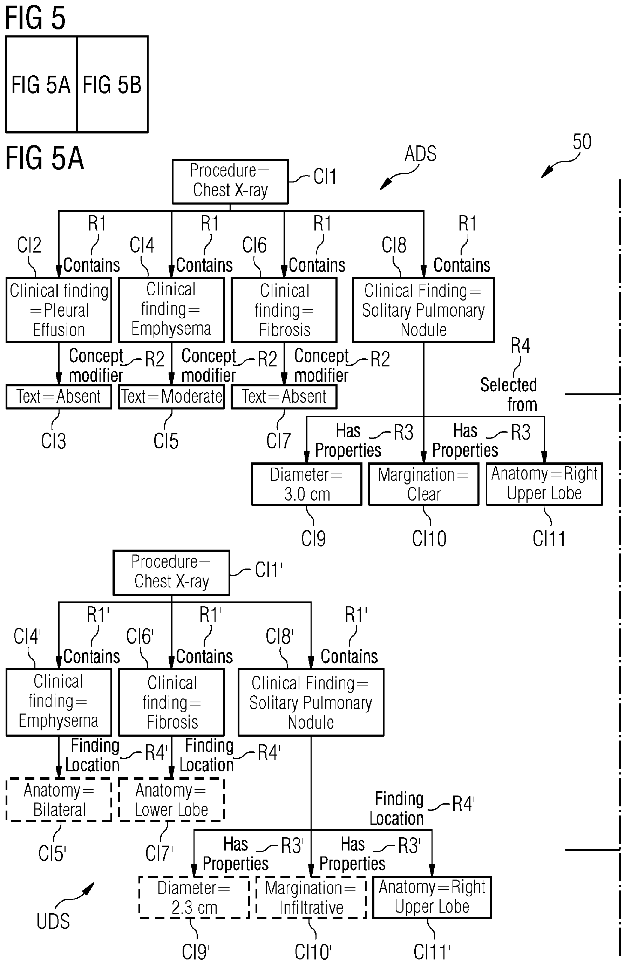

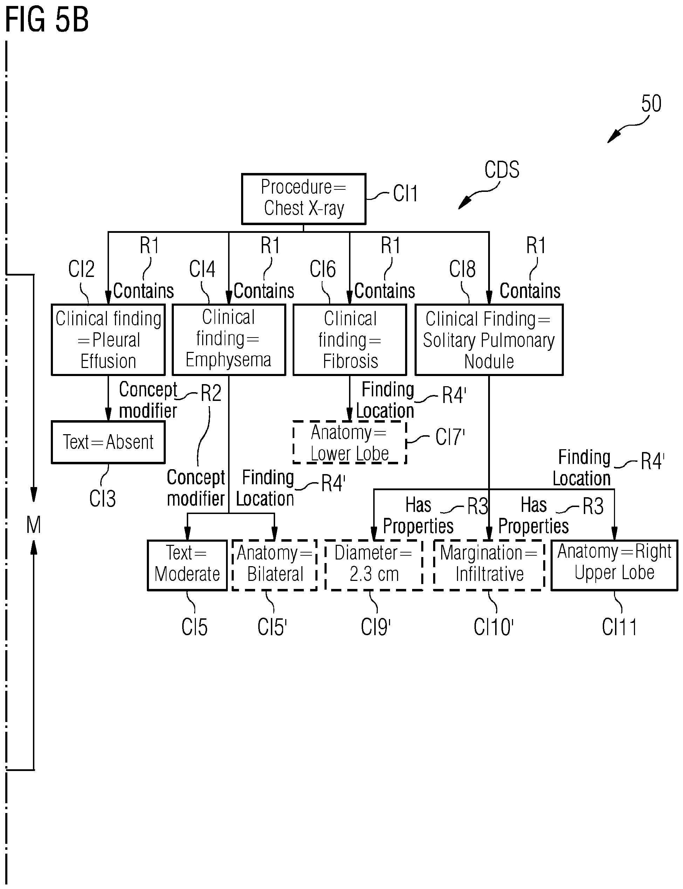

As can be taken from FIG 2 and FIG 4 and also later illustrated in FIG 5, some of the corresponding concept items of the AI-generated DICOM SR object ADS in FIG 2 and the DICOM SR object UDS generated based on user-generated data in FIG 4 are the same and other differ from each other. For example, CI1 = CI1' = Procedure = Chest X-ray, CI4 = CI4' = Clinical finding = Emphysema. However, CI5 = Text = Moderate, but CI5' = Anatomy = Bilateral, CI7 = Text = Absent, but CI7' = Anatomy = Lower Lobe, CI9 = Diameter = 3.0 cm, but CI9' = Diameter = 2.3 cm.

The relationship items R1', R3', R4' used in FIG 4 are illustrated in an explicit way in the following table:

R1' contains
R3' has properties
R4' finding location.

Some of the corresponding relationships in FIG 2 und FIG 4 are the same as for example "contains" and "has properties" and some relationships as R4 "selected from" and R4' "finding location", are very similar. However, the relationship R2 "concept modifier" is only used for the AI-generated DICOM SR object ADS in FIG 2.

In FIG 5, which is divided in FIG 5A and FIG 5B, an illustration 50 of a merging process M between an AI-based generated DICOM SR object ADS (in FIG 5A, upper part, already shown in FIG 2) and a DICOM SR object UDS (in FIG 5A, lower part, already shown in FIG 4) generated based on user-generated data is depicted. The result of the merging process M is a final composite DICOM SR object CDS (depicted in FIG 5B), for example usable for archival in patient records. The final composite DICOM SR object CDS is represented as a directed acyclic graph with vertices as the concept items and edges represented by relationships. A simple realization of such a graph merge and edit can be rule based.

Details of the AI-based generated DICOM SR object ADS and the DICOM SR object UDS based on user-generated data have been described in context with FIG 2 and FIG 4 and therefore, a description of these details is omitted for FIG 5A.

The concept items CI1, CI2, ..., CI11 of the final composite DICOM SR object CDS shown in FIG 5B are illustrated in an explicit way in the following table:

CI1 Procedure = Chest X-ray
CI2 Clinical finding = Pleural Effusion
CI3 Text = Absent
CI4 Clinical finding = Emphysema
CI5 Text = Moderate
CI5' Anatomy = Bilateral
CI6 Clinical finding = Fibrosis
CI7' Anatomy = Lower Lobe
CI8 Clinical Finding = Solitary Pulmonary Nodule

CI9' Diameter = 2.3 cm
CI10' Margination = Infiltrative
CI11 Anatomy = Right Upper Lobe.

As can be taken from FIG 5A and FIG 5B, the concept item CI5' differs from the concept item CI5 and both CI5 and CI5' are received in the final composite DICOM SR object CDS, since they are complementary and not contradictory. CI7' differs from CI7 and CI7' replaces CI7 in the resulting final composite DICOM SR object CDS, since CI7 and CI7' are contradictory. CI9' differs from CI9 and CI9' replaces CI9 in the resulting final composite DICOM SR object CDS, since CI9 and CI9' are contradictory. CI10' differs from CI10 and CI10' replaces CI10 in the resulting final composite DICOM SR object CDS, since CI10 and CI10' are contradictory. If there is no contradiction between the concept items generated based on the user-generated data and AI-based generated concept items as it is the case for CI5 and CI5', both concept items are received in the resulting final composite DICOM SR object CDS. If there is a contradiction between a concept item generated based on the user-generated data and an AI-generated concept item as it is the case for CI7, CI9, CI10 and CI7', CI9' and CI10', the concept item generated based on user-generated data replaces the AI-generated concept item.

The relationships R1, R2, R3, R4' are illustrated in an explicit way in the following table:

R1 contains
R2 concept modifier
R3 has properties
R4' finding location.

Similarly, if there is a contradiction between relationships R1',..., R4' generated based on the user-generated data and the AI-generated relationships R1,..., R4, as it is the case for R2 and R4' (R4' "finding location" CI7' Anatomy = Lower Lobe replaces R2 "concept modifier" CI7 Text = Absent) or R4' and R4, the relationship generated based on user-generated data replaces the AI-generated relationships.

Hence, results based on user-generated data always supersede AI-generated results in times of conflict. If a conflict exists, the AI-generated result is overwritten by human generated DICOM SR concept items, i.e. DICOM SR concept items generated based on user-generated data. If there is no conflict, the graph nodes, i.e. concept items, and the relationships are merged and both concept items or relationships are received in the resulting structured medical data object, i.e. in particular the final composite DICOM SR object CDS. Alternatively, more advanced graph linking approaches can also be implemented such as hierarchical clustering, graph neural networks, etc. Once the final composite DICOM SR object CDS in form of a composite graph is generated, it can be archived in an electronic health record EHR (shown in FIG 1) of the patient for archival purposes.

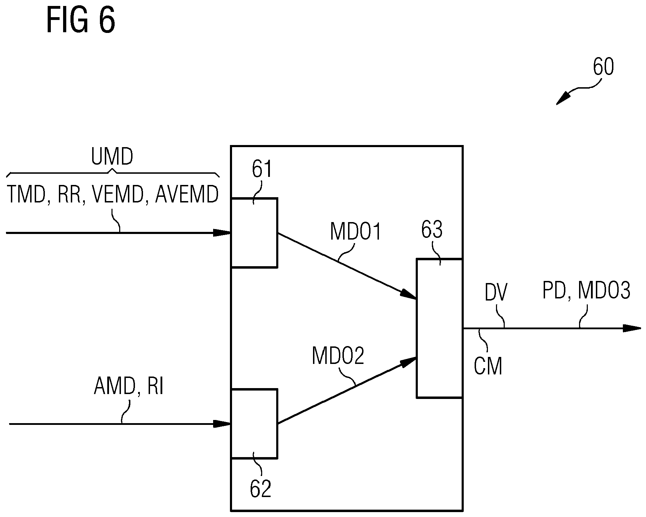

In FIG 6, a schematic view on a protocol data generation device 60 according to an embodiment of the invention is shown.

The protocol data generation device 60 includes an extraction unit 61. The extraction unit 61 is arranged to generate a first structured medical data object MDO1, for example a DICOM SR object, based on user-generated electronic medical data UMD. As already mentioned, the user-generated electronic medical data UMD may include text-based electronic medical data TMD and in particular a user-generated radiological report RR of a free text related to medical image data of a patient. The user-generated electronic medical data UMD may also include electronic medical data generated by voice VEMD or audio-visually generated electronic medical data AVEMD. Electronic medical data generated by voice VEMD include a radiology report generated by voice and audio-visually generated electronic medical data AVEMD include a video-based radiology report.

Part of the protocol data generation device 60 is also a processing unit 62 for generating a second structured medical data object MDO2. The second structured medical data object MDO2 is generated by automatically determining findings and annotations based in the above-mentioned medical image data of a radiology image RI, which is a special type of automatically created electronic medical data AMD. The determination of findings and annotations is realized using an AI-based processing algorithm.

Further, the protocol data generation device 60 includes a data generation unit 63 for generating a merged third structured medical data object MDO3 and emits protocol data PD including a merged third structured medical data object MDO3. The merged third structured medical data object MDO3 is generated based on the first structured medical data object MDO1 and on the second structured medical data object MDO2. The merged third structured medical data object MDO3 includes information about deviations DV between the first and second structured medical data object MDO1, MDO2. Also complements CM, a special type of deviations DV, are indicated by the merged third structured medical data object MDO3.

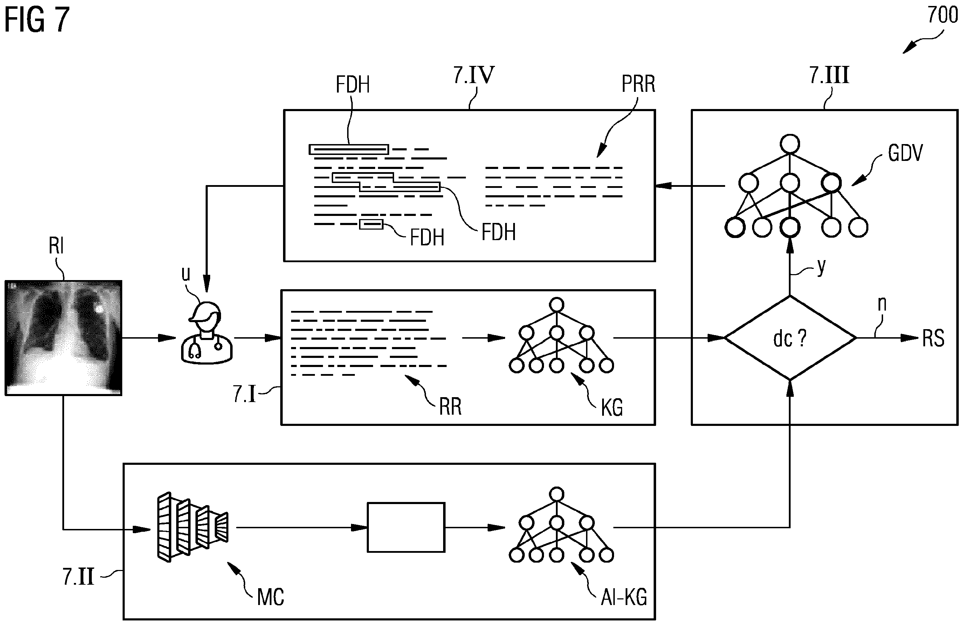

In FIG 7, a schematic illustration of an AI-based explainable peer review of a radiology report RR which is generated by a user U based on a radiology image RI is shown.

In step 7.1, the incoming radiology report RR is parsed and mapped to a radiology knowledge graph KG representation. In parallel, in step 7.II, the radiology image RI is read by a multi-class classifier MC or an AI-based post-processing algorithm (not shown in FIG 7) that maps its results to a similar AI-generated radiology knowledge graph AI-KG.

In step 7.III, the two radiology knowledge graphs KG, AI-KG are compared using a graph similarity network which outputs a similarity measure. If there is a difference dc between the compared radiology knowledge graphs KG, AI-KG, which is symbolized in FIG 7 with "y", a difference graph GDV is generated. Further, graph explanation and visualization methods are used to highlight the difference. If there is no difference dc between the compared knowledge graphs KG, AI-KG, which is symbolized in FIG 7 with "n", the radiology report RR is signed off using a sign symbol RS.

Then, in step 7.IV the difference graph GDV is converted to a human readable text using a report generation deep recurrent model. This is presented to the radiologist, i.e. the user U, as a peer-review report PRR for consideration prior to report sign off. The peer-review report PRR includes highlighted findings FDH with differences as depicted in the left sub portion of the field representing step 7.IV in FIG 7.

A report generation deep recurrent model is described in the following documents:

Hou, B., Kaissis, G., Summers, R.M. and Kainz, B., 2021, September. RATCHET: Medical Transformer for Chest X-ray Diagnosis and Reporting. In International Conference on Medical Image Computing and Computer-Assisted Intervention (pp. 293-303). Springer, Cham and

Banerjee, I., Ling, Y., Chen, M.C., Hasan, S.A., Langlotz, C.P., Moradzadeh, N., Chapman, B., Amrhein, T., Mong, D., Rubin, D.L. and Farri, O., 2019. Comparative effectiveness of convolutional neural network (CNN) and recurrent neural network (RNN) architectures for radiology text report classification. Artificial intelligence in medicine, 97, pp.79-88.

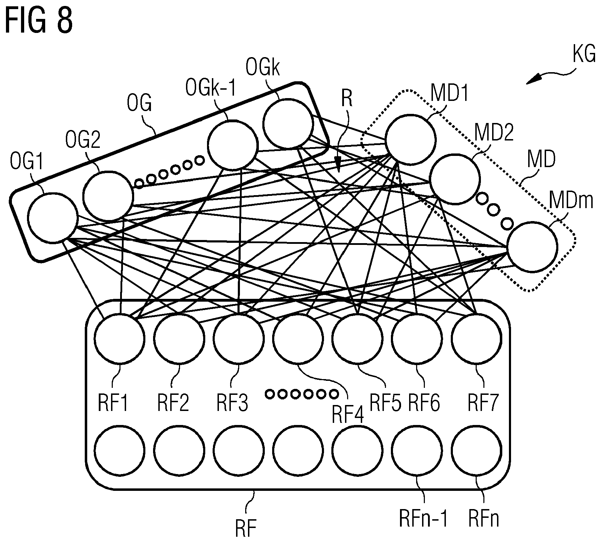

In FIG 8, a schematic view on a representation of a radiology knowledge graph KG is shown. In this particular realization, the entities are grouped according to the concepts of organs OG, modalities MD and the corresponding radiographic findings RF. For example different entities or concept items OG1, OG2, ..., OGk represent different types of organs OG. Different concept items MD1, MD2, ..., MDm represent different types of modalities MD and different concept items RF1, RF2, ..., RFn represent different types of radiology findings RF.

The represented organs OG are shown in the following table:

| OG1 | Chest |
| OG2 | Breast |
| OGk-1 | Brain |
| OGk | Shoulder. |

The represented modalities MD are shown in the following table:

| MD1 | CT |
| MD2 | MRI |

(continued)

| | |
|---|---|
| MDm | X-ray. |

The represented radiographic findings RF are shown in the following table:

| | |
|---|---|
| RF1 | Lesion |
| RF2 | Phneumothorax |
| RF3 | Effusion |
| RF4 | Pneumonia |
| RF5 | Fracture |
| RF6 | Calcification |
| RF7 | Inflammation. |

Representing radiological concepts in the form of radiology knowledge graph data structures allows for dedicated feature learning of the individual concepts and modelling relationships R between them. The radiology knowledge graph KG shown in FIG 8 is implemented as a global radiology knowledge graph KG including all combinations of relations between organs OG, modalities MD and the corresponding radiographic findings RF. Such a global radiology knowledge graph KG is difficult to explore due to its large size and due to a lack of clinical relevance of a number of nodes to the case at hand.

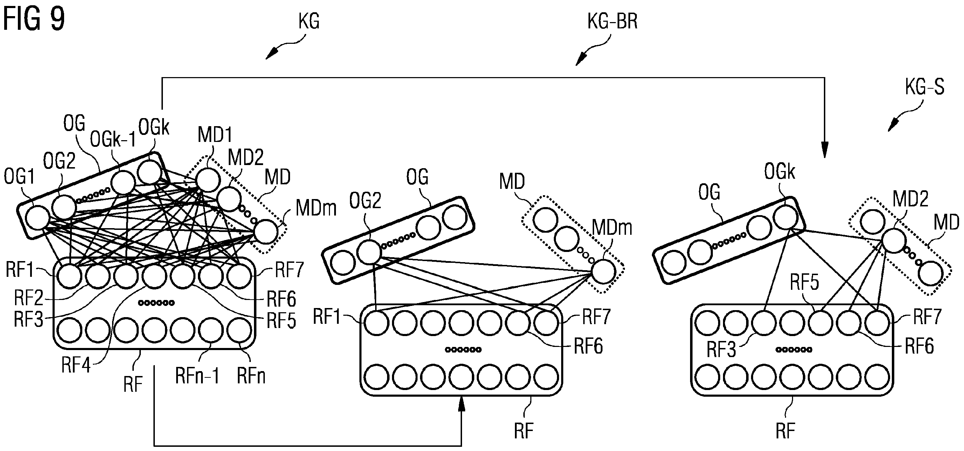

In FIG 9, a reduction of a global radiology knowledge graph KG to relevant subgraphs KG-BR, KG-S is illustrated. In the middle of FIG 9, a knowledge graph KG-BR representing a reduction to an X-ray image of a breast is illustrated and on the right side of FIG 9, a knowledge graph KG-S representing a reduction to an MR-image of a shoulder is represented. In case of the knowledge graph KG-BR representing a reduction to an X-ray image only the breast OG2 is examined using an X-ray imaging system MDm (abbreviated by X-ray) and only the relevant findings, i.e. a lesion RF1, a calcification RF6 and an inflammation RF7 are linked with the mentioned organ OG2 and modality MDm. In case of the knowledge graph KG-S representing a reduction to an MR-image of a shoulder, only the shoulder OGk is examined using an MR imaging system MD2 (abbreviated by MRI) and only the relevant findings, i.e. an effusion RF3, a fracture RF5, a calcification RF6 and an inflammation RF7 are linked with the mentioned organ OGk and modality MD2.

For reduction, the correct combination of attributes, i.e. values and concept items inferable from image meta data, in particular from DICOM meta data, is used to extract the right semantically relevant subgraph KG-BR, KG-S from the global radiology knowledge graph KG.

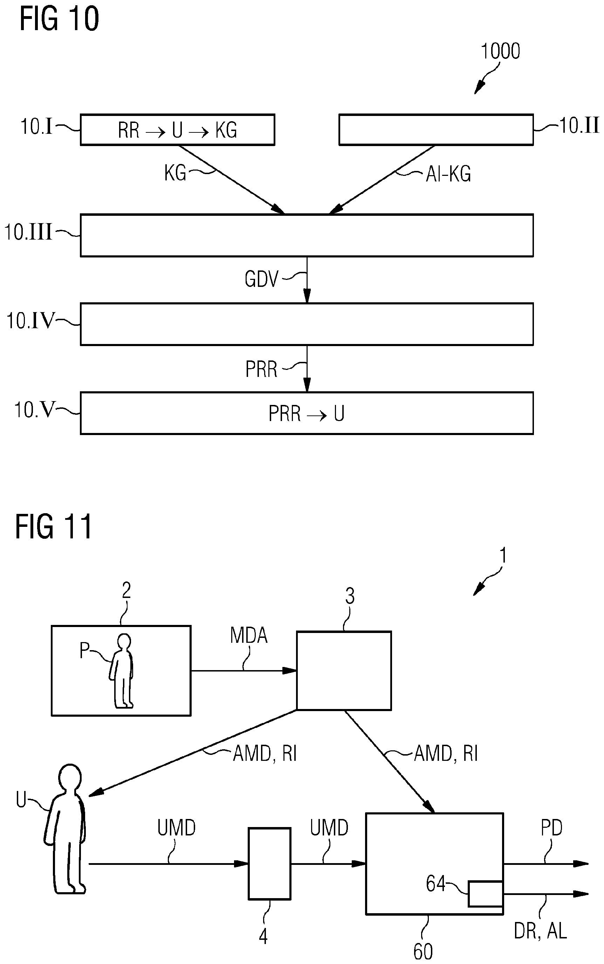

In FIG 10, a flow chart diagram 1000 is shown, illustrating a special embodiment of the method for generating protocol data of a specific medical process according to the invention, wherein an AI-based peer review of a text-based radiology report RR is generated, also named AI-based peer review method.

In step 10.I, based on an appropriately selected subgraph structure, the radiology report RR is parsed to extract relationships that have been reported as present or absent or not-reported.

The extraction of a dependency graph, i.e. a radiology knowledge graph KG from a textual report, is realized by tokenization approaches to identify key parts of speech, named entities such as anatomy, radiographic findings and differential diagnosis. Tokenizing, dependency parsing and mapping to the knowledge graph KG by matching detected named entities and relationships, i.e. "present", "absent", "not-reported" or "uncertain", into edge weights can be accomplished by using tools such as Stanford CoreNLP.

In step 10.11, the generation of an AI-generated radiology knowledge graph AI-KG is implemented using computer aided detection tools. After having received an appropriately selected subgraph structure, vertices are discovered and are connected based on relationships also named edges between the vertices. For example, a number of key features that can be extracted using computer aided detection or diagnostic tools are captured as edge weights for determining the relationships.

The relationships are set based on different rules. Such a rule can include the order that if radiographic findings cooccur in the same anatomy, then those nodes are linked with a bidirectional connection. Alternatively, a relationship can also be established based on conditional probabilities estimated using AI-based methods for computer aided detection and diagnosis of particular radiographic findings in body regions of a patient.

In step 10.III, a graph matching to generate a difference graph is realized. The two input graphs KG, AI-KG are used for comparison. Based on one radiology knowledge graph KG reported by the radiologist and one radiology knowledge graph AI-KG generated by an artificial intelligence, it can be performed graph matching to find all the elements that differ significantly between the two radiology knowledge graphs KG, AI-KG. It can be started by randomly parsing the AI-generated radiology knowledge graph AI-KG into smaller sub-subgraphs and trying to find a match of the sub-subraphs to the subgraph KG reported by the radiologist using the Hungarian algorithm or variants of it. This algorithm is described in https://en.wikipedia.org/wiki/hungarian-algorithm. This can be accomplished using methods such as SimRank, similarity flooding, etc. If a match is found, then the matched subsubgraph is deemed as information already reported. If a match is not found, this is added to the difference graph GDV.

In step 10.IV, the difference graph GDV generated in step 10.III is converted into human readable clinical text, i.e. a peer review report PRR, using templates with named entities as placeholders. An alternative to rule based methods is to employ deep learning models such with a graph convolution encoder feeding to a text generation recurrent neural network-based decoder.

In step 10.V, the peer review report PRR is displayed to the user.

As already mentioned, once trained, the explainable AI-based peer review method can be deployed as a micro-service that is triggered in the backend as soon as a particular case is sent to the radiology worklist.

In FIG 11, a medical process system 1 according to an embodiment of the invention is illustrated. The medical process system 1, in particular a medical imaging system, performs a specific medical process. The medical process system 1 includes a process unit 2 for applying the specific medical process to a patient P. The specific medical process also comprises a measurement process for generating measurement data MDA, for example raw data of an X-ray imaging process.

The generated measurement data MDA are sent to a documentation unit 3 which is also a part of the medical process system 1. The documentation unit 3, for example a reconstruction unit, is arranged to generate automatically created electronic medical data AMD documenting the specific medical process, i.e. the medical imaging. For example, the automatically created electronic medical data AMD include a radiology image RI.

Further, the medical process system 1 also includes a user interface 4 for receiving user-generated electronic medical data UMD created by a user U documenting the specific medical process. For example, the user-generated electronic medical data UMD include user-generated annotations generated based on the radiology image RI by the user U.

Furthermore, the medical process system 1 includes a protocol data generation device 60 according to an embodiment of the invention as it is described in context with FIG 6.

The protocol data generation device 60 generates protocol data PD based on the received user-generated electronic medical data UMD and the automatically created electronic medical data AMD. As illustrated in FIG 1 to FIG 5 and FIG 7 to FIG 9, the protocol data generation device 60 generates as an intermediate step a first structured medical data object MDO1 and a second structured medical data object MDO2 (not shown in FIG 11) based on the received user-generated electronic medical data UMD and the automatically created electronic medical data AMD.

The protocol data generation device 60 can optionally include a deviation detection reaction unit 64 for performing a deviation detection reaction DR, for example an alert AL, based on detected deviations between the first structured medical data object MDO1 and the second structured medical data object MDO2.

The above descriptions are merely preferred embodiments of the present disclosure, but not intended to limit the present disclosure, and any modifications, equivalent replacements, improvements, etc. made within the spirit and principle of the present disclosure should be included within the scope of protection of the present disclosure.

Further, the use of the undefined article "a" or "one" does not exclude that the referred features can also be present several times. Likewise, the term "unit" or "device" does not exclude that it consists of several components, which may also be spatially distributed.

## Claims

1. Method for generating protocol data (PD, MDO3, CDS, PRR) of a specific medical process (SMP), wherein the specific medical process (SMP) comprises at least one measurement process, preferably a radiological imaging process,
   wherein the method comprises the steps of:

   - generating a first structured medical data object (MDO1, UDS, KG) based on user-generated electronic medical data (UMD, TMD, RR, VEMD, AVEMD), wherein the user-generated electronic medical data (UMD, TMD, RR, VEMD, AVEMD) are created by a user (U) documenting the specific medical process (SMP),
   - generating a second structured medical data object (MDO2, ADS, AI-KG) by applying an automatic processing algorithm (APA, AIM, MC) to automatically created electronic medical data (AMD, RI), wherein the electronic medical data (AMD, RI) refer to the specific medical process (SMP),
   - generating the protocol data (PD, MDO3, CDS, PRR) based on the first structured medical data object (MDO1, UDS, KG) and on the second structured medical data object (MDO2, ADS, AI-KG), wherein the first and second structured medical data objects (MDO1, UDS, KG, MDO2, ADS, AI-KG) are compared to determine deviations (DV, CM).

2. Method according to claim 1, wherein if deviations (DV, CM) are determined, a deviation detection reaction (DR) is performed, whereby preferably the deviation detection reaction (DR) comprises at least one of the following actions:

   - generating an alert (AL) output for a user (U),
   - generating amended protocol data (PD, MDO3, CDS, PRR),
   - generating protocol data (PD, MDO3, CDS, PRR) including marks indicating deviations (DV, CM),
   - including measurement data with marked deviations (DV, CM) into the protocol data (PD, MDO3, CDS, PRR).

3. Method according to claim 1 or 2, wherein the generated protocol data (PD, MDO3, CDS, PRR) comprise a third structured medical data object (MDO3, CDS, GDV).

**4.** Method according to any of the preceding claims, wherein the first and the second structured medical data object (MDO1, UDS, KG, MDO2, ADS, AI-KG) comprise the same data structure.

**5.** Method according to any of the preceding claims, wherein the user-generated electronic medical data (UMD, TMD, RR, VEMD, AVEMD) comprise at least one of:

     - text-based electronic medical data (TMD),
     - electronic medical data (VEMD) generated by voice,
     - audio-visually generated electronic medical data (AVEMD).

**6.** Method according to any of the preceding claims, wherein

     - the user-generated electronic medical data (UMD, TMD, RR, VEMD, AVEMD) include a radiological report (RR) compirising free text,
     - the automatically created electronic medical data (AMD, RI) include medical image data (RI) on which the radiological report (RR) is based, and
     - the automatic processing algorithm (APA, AIM) comprises an image processing algorithm which is applied to the medical image data (RI).

**7.** Method according to any of the preceding claims, wherein the structured medical data objects (MDO1, UDS, KG, MDO2, ADS, AI-KG) comprise a graph structure.

**8.** Method according to any of the preceding claims, wherein the structured medical data objects (MDO1, UDS, MDO2, ADS, MDO3, CDS) comprise one of the following types:

     - a DICOM SR object (UDS, ADS, CDS),
     - a HL7 object,
     - a FHIRcast object,
     - an XML object.

**9.** Method according to any of claims 5 to 8, wherein if the user-generated electronic medical data (UMD, TMD, RR) comprise text-based electronic medical data (TMD, RR), the step of generating a first structured medical data object (MDO1, UDS, KG) is implemented by content extraction, comprising the steps of:

     - parsing the text-based electronic medical data (TMD, RR) and
     - generating the first structured medical data object (MDO1, UDS, KG) based on the parsed text-based electronic medical data (TMD, RR), wherein the parsed text-based electronic medical data (TMD, RR) are grouped into structures which form the first structured medical data object (MDO1, UDS, KG) .

**10.** Method according to claim 9, wherein the parsing step includes at least one of:

     - generating disambiguated terms (DT) by clinical word-sense disambiguation for acronyms and abbreviations in the text-based electronic medical data (TMD, RR),
     - semantic mapping of semantically similar terms in the text-based electronic medical data (TMD, RR) to standardized terms (ST),
     - optionally machine translation (MT) of the text-based electronic medical data (TMD, RR),
     - clinical named entity (NE) recognition to locate and classify clinical named entities (NE) mentioned in the text-based electronic medical data (TMD, RR) into pre-defined categories.

**11.** Method according to claim 10, wherein the step of generating the first structured medical data object (MDO1, UDS, KG) includes at least one of:

     - mapping recognized clinical named entities (NE) to concept items (CI1', CI4',..., CI11'),
     - extracting relationships (R1', R3', R4') between each concept item (CI1', CI4',.., CI11'),
     - constructing a first structured medical data object (MDO1, UDS, KG) based on the concept items (CI1', CI4',..., CI11') and the extracted relationships (R1', R3', R4').

**12.** Protocol data generation device (60), comprising:

     - an extraction unit (61) for generating a first structured medical data object (MDO1, UDS, KG) based on user-generated electronic medical data (UMD, TMD, RR, VEMD, AVEMD), which user-generated electronic medical data (UMD, TMD, RR, VEMD, AVEMD) are created by a user (U) documenting a specific medical process (SMP) comprising at least one measurement process,
     - a processing unit (62) for generating a second structured medical data object (MDO2, ADS, AI-KG) by applying an automatic processing algorithm (APA, AIM) to automatically created electronic medical data (AMD, RI), which electronic medical data (AMD) refer to the specific medical process (SMP),
     - a data generation unit (63) for generating the protocol data (PD, MDO3, CDS, PRR) based on the first structured medical data object (MDO1, UDS, KG) and on the second structured medical data object (MDO2, ADS, AI-KG), wherein the first and second structured medical data objects (MDO1, UDS, KG, MDO2, ADS, AI-KG) are compared to determine deviations (DV, CM).

**13.** A medical process system (1), comprising:

- a process unit (2) for applying a specific medical process (SMP) which comprises at least one measurement process to a patient (P),
- a documentation unit (3) for generating automatically created electronic medical data (AMD, RI) documenting the specific medical process (SMP),
- a user interface (4) for receiving user-generated electronic medical data (UMD, TMD, RR, VEMD, AVEMD) created by a user (U) documenting the specific medical process (SMP),
- a protocol data generation device (60) according to claim 12.

**14.** A computer program product comprising instructions which, when the program is executed by a computer, cause the computer to carry out the steps of the method according to any of the claims 1 to 11.

**15.** A computer-readable storage medium comprising instructions which, when executed by a computer, cause the computer to carry out the steps of the method according to any of the claims 1 to 11.

**Amended claims in accordance with Rule 137(2) EPC.**

**1.** Method for generating protocol data (PD, MDO3, CDS, PRR) of a specific medical process (SMP), wherein the specific medical process (SMP) comprises at least one measurement process, preferably a radiological imaging process, wherein the method comprises the steps of:

- generating a first structured medical data object (MD01, UDS, KG) based on user-generated electronic medical data (UMD, TMD, RR, VEMD, AVEMD), wherein the user-generated electronic medical data (UMD, TMD, RR, VEMD, AVEMD) are created by a user (U) documenting the specific medical process (SMP),
- generating a second structured medical data object (MDO2, ADS, AI-KG) by applying an automatic processing algorithm (APA, AIM, MC) to automatically created electronic medical data (AMD, RI), wherein the electronic medical data (AMD, RI) refer to the specific medical process (SMP), wherein the first and the second structured medical data object (MD01, UDS, KG, MDO2, ADS, AI-KG) comprise the same data structure,

- generating the protocol data (PD, MDO3, CDS, PRR) based on the first structured medical data object (MD01, UDS, KG) and on the second structured medical data object (MDO2, ADS, AI-KG), wherein the first and second structured medical data objects (MD01, UDS, KG, MDO2, ADS, AI-KG) are compared to determine deviations (DV, CM).

**2.** Method according to claim 1, wherein if deviations (DV, CM) are determined, a deviation detection reaction (DR) is performed, whereby preferably the deviation detection reaction (DR) comprises at least one of the following actions:

- generating an alert (AL) output for a user (U),
- generating amended protocol data (PD, MDO3, CDS, PRR),
- generating protocol data (PD, MDO3, CDS, PRR) including marks indicating deviations (DV, CM),
- including measurement data with marked deviations (DV, CM) into the protocol data (PD, MDO3, CDS, PRR).

**3.** Method according to claim 1 or 2, wherein the generated protocol data (PD, MDO3, CDS, PRR) comprise a third structured medical data object (MDO3, CDS, GDV).

**4.** Method according to any of the preceding claims, wherein the user-generated electronic medical data (UMD, TMD, RR, VEMD, AVEMD) comprise at least one of:

- text-based electronic medical data (TMD),
- electronic medical data (VEMD) generated by voice,
- audio-visually generated electronic medical data (AVEMD).

**5.** Method according to any of the preceding claims, wherein

- the user-generated electronic medical data (UMD, TMD, RR, VEMD, AVEMD) include a radiological report (RR) compirising free text,
- the automatically created electronic medical data (AMD, RI) include medical image data (RI) on which the radiological report (RR) is based, and
- the automatic processing algorithm (APA, AIM) comprises an image processing algorithm which is applied to the medical image data (RI).

**6.** Method according to any of the preceding claims, wherein the structured medical data objects (MD01, UDS, KG, MDO2, ADS, AI-KG) comprise a graph structure.

7. Method according to any of the preceding claims, wherein the structured medical data objects (MD01, UDS, MDO2, ADS, MDO3, CDS) comprise one of the following types:

- a DICOM SR object (UDS, ADS, CDS),
- a HL7 object,
- a FHIRcast object,
- an XML object.

8. Method according to any of claims 4 to 7, wherein if the user-generated electronic medical data (UMD, TMD, RR) comprise text-based electronic medical data (TMD, RR), the step of generating a first structured medical data object (MD01, UDS, KG) is implemented by content extraction, comprising the steps of:

- parsing the text-based electronic medical data (TMD, RR) and
- generating the first structured medical data object (MD01, UDS, KG) based on the parsed text-based electronic medical data (TMD, RR), wherein the parsed text-based electronic medical data (TMD, RR) are grouped into structures which form the first structured medical data object (MD01, UDS, KG).

9. Method according to claim 8, wherein the parsing step includes at least one of:

- generating disambiguated terms (DT) by clinical word-sense disambiguation for acronyms and abbreviations in the text-based electronic medical data (TMD, RR),
- semantic mapping of semantically similar terms in the text-based electronic medical data (TMD, RR) to standardized terms (ST),
- optionally machine translation (MT) of the text-based electronic medical data (TMD, RR),
- clinical named entity (NE) recognition to locate and classify clinical named entities (NE) mentioned in the text-based electronic medical data (TMD, RR) into pre-defined categories.

10. Method according to claim 9, wherein the step of generating the first structured medical data object (MD01, UDS, KG) includes at least one of:

- mapping recognized clinical named entities (NE) to concept items (CI1', CI4',..., CI11'),
- extracting relationships (R1', R3', R4') between each concept item (CI1', CI4',..., CI11'),
- constructing a first structured medical data object (MD01, UDS, KG) based on the concept items (CI1', CI4', ..., CI11') and the extracted relationships (R1', R3', R4').

11. Protocol data generation device (60), comprising:

- an extraction unit (61) for generating a first structured medical data object (MD01, UDS, KG) based on user-generated electronic medical data (UMD, TMD, RR, VEMD, AVEMD), which user-generated electronic medical data (UMD, TMD, RR, VEMD, AVEMD) are created by a user (U) documenting a specific medical process (SMP) comprising at least one measurement process,
- a processing unit (62) for generating a second structured medical data object (MDO2, ADS, AI-KG) by applying an automatic processing algorithm (APA, AIM) to automatically created electronic medical data (AMD, RI), which electronic medical data (AMD) refer to the specific medical process (SMP), wherein the first and the second structured medical data object (MD01, UDS, KG, MDO2, ADS, AI-KG) comprise the same data structure,
- a data generation unit (63) for generating the protocol data (PD, MDO3, CDS, PRR) based on the first structured medical data object (MD01, UDS, KG) and on the second structured medical data object (MDO2, ADS, AI-KG), wherein the first and second structured medical data objects (MD01, UDS, KG, MDO2, ADS, AI-KG) are compared to determine deviations (DV, CM).

12. A medical process system (1), comprising:

- a process unit (2) for applying a specific medical process (SMP) which comprises at least one measurement process to a patient (P),
- a documentation unit (3) for generating automatically created electronic medical data (AMD, RI) documenting the specific medical process (SMP),
- a user interface (4) for receiving user-generated electronic medical data (UMD, TMD, RR, VEMD, AVEMD) created by a user (U) documenting the specific medical process (SMP),
- a protocol data generation device (60) according to claim 11.

13. A computer program product comprising instructions which, when the program is executed by a computer, cause the computer to carry out the steps of the method according to any of the claims 1 to 10.

14. A computer-readable storage medium comprising instructions which, when executed by a computer, cause the computer to carry out the steps of the method according to any of the claims 1 to 10.

# FIG 1

EP 4 239 642 A1

# FIG 2

# FIG 3

300

3.I

|    | DT |
|    |

3.II

|    | ST |

3.III

|    | MT |

3.IV

|    | NE |

3.V

|    | CI |

3.VI    CI, R → UDS

## FIG 4

RR

400

Chest X-ray image:

Bilateral emphysematous again noted and lower lobe fibrotic changes. Solitary Pulmonary Nodule measuring 2.3 cm diameter with infiltrative margins noted in right upper lobe.

4.II

UDS

```
                                    ┌─────────────┐
                                    │ Procedure=  │ ─ CI1'
                                    │ Chest X-ray │
                                    └─────────────┘
              R1'              R1'              R1'
      CI4'  Contains   CI6'  Contains   CI8'  Contains
      ┌──────────┐    ┌──────────┐    ┌──────────────┐
      │ Clinical │    │ Clinical │    │ Clinical     │
      │ finding= │    │ finding= │    │ Finding=     │
      │ Emphysema│    │ Fibrosis │    │ Solitary     │
      └──────────┘    └──────────┘    │ Pulmonary    │
                                      │ Nodule       │
                                      └──────────────┘
       Finding   R4'   Finding   R4'
       Location        Location                    R4'
```

4.I

4.II

```
      ┌──────────┐    ┌──────────┐          Finding Location
      │ Anatomy= │    │ Anatomy= │
      │ Bilateral│    │ Lower Lobe│
      └──────────┘    └──────────┘
      CI5'             CI7'
                                    Has  R3'    Has  R3'
                                  Properties  Properties
```

CI

```
┌────────────┐        ┌──────────┐        ┌──────────┐
│ Procedure= │ ─ CI1' │ Anatomy= │ ─ CI7' │ Diameter=│ ─ CI9'
│ Chest X-ray│        │ Lower Lobe│        │ 2.3 cm   │
└────────────┘        └──────────┘        └──────────┘

┌────────────┐        ┌──────────┐        ┌────────────┐
│ Anatomy=   │ ─ CI5' │ Clinical │ ─ CI6' │ Margination=│ ─ CI10'
│ Bilateral  │        │ finding  │        │ Infiltrative│
└────────────┘        │ =Fibrosis│        └────────────┘
                      └──────────┘
┌────────────┐        ┌──────────┐        ┌──────────┐
│ Clinical   │ ─ CI4' │ Clinical │ ─ CI8' │ Anatomy= │ ─ CI11'
│ finding=   │        │ finding  │        │ Right    │
│ Emphysema  │        │ =Solitary│        │ Upper    │
└────────────┘        │ Pulmonary│        │ Lobe     │
                      │ Nodule   │        └──────────┘
                      └──────────┘
```

```
┌──────────┐    ┌────────────┐    ┌────────────┐
│ Diameter=│    │ Margination=│   │ Anatomy=Right│
│ 2.3 cm   │    │ Infiltrative│   │ Upper Lobe  │
└──────────┘    └────────────┘    └────────────┘
   CI9'            CI10'              CI11'
```

EP 4 239 642 A1

# FIG 5

| FIG 5A | FIG 5B |
|---|---|

# FIG 5A

ADS

50

Procedure=
Chest X-ray — CI1

R1          R1          R1          R1

CI2         CI4         CI6         CI8

Contains    Contains    Contains    Contains

Clinical finding   Clinical      Clinical       Clinical Finding=
=Pleural           finding=      finding=       Solitary Pulmonary
Effusion           Emphysema     Fibrosis       Nodule

R4

Concept —R2   Concept —R2   Concept —R2         Selected
modifier      modifier      modifier            from

Text=Absent   Text=Moderate   Text=Absent

CI3           CI5             CI7

Has —R3        Has —R3
Properties     Properties

Diameter=     Margination=    Anatomy=Right
3.0 cm        Clear           Upper Lobe

CI9           CI10            CI11

Procedure=
Chest X-ray — CI1'

R1'          R1'          R1'

CI4'         CI6'         CI8'

Contains     Contains     Contains

Clinical       Clinical       Clinical Finding=
finding=       finding=       Solitary Pulmonary
Emphysema      Fibrosis       Nodule

Finding —R4'   Finding —R4'
Location       Location

Anatomy=       Anatomy=                    Finding —R4'
Bilateral      Lower Lobe                  Location

CI5'           CI7'

Has —R3'       Has —R3'
Properties     Properties

UDS            Diameter=      Margination=    Anatomy=Right
               2.3 cm         Infiltrative    Upper Lobe

CI9'           CI10'          CI11'

# FIG 5B

50

CDS

```
                    Procedure=
                    Chest X-ray        CI1

        R1            R1            R1            R1
   CI2          CI4          CI6          CI8
      Contains     Contains     Contains        Contains

Clinical finding   Clinical      Clinical      Clinical Finding=
=Pleural          finding=      finding=      Solitary Pulmonary
Effusion          Emphysema     Fibrosis      Nodule

   Concept   R2                 Finding   R4'
   modifier                     Location

Text=Absent                     Anatomy=      CI7'
                                Lower Lobe

   CI3
                                                              Finding    R4'
            Concept   Finding   R4'                           Location
            modifier  Location
                                         Has    R3      Has    R3
                                      Properties      Properties

Text=      Anatomy=  Diameter=  Margination=  Anatomy=Right
Moderate   Bilateral  2.3 cm     Infiltrative  Upper Lobe

CI5        CI5'       CI9'       CI10'         CI11
```

M

## FIG 6

60

UMD

TMD, RR, VEMD, AVEMD

61

MD01

63

DV    PD, MD03

CM

AMD, RI

MD02

62

FIG 7

EP 4 239 642 A1

FIG 8

FIG 9

EP 4 239 642 A1

## FIG 10

1000

10.I — | RR → U → KG |    |    | — 10.II

KG    AI-KG

10.III — | |

GDV

10.IV — | |

PRR

10.V — | PRR → U |

## FIG 11

1

2    3

P    MDA

AMD, RI    AMD, RI

U

UMD    UMD    PD

4    64

60    DR, AL

Europäisches
Patentamt

European
Patent Office

Office européen
des brevets

# EUROPEAN SEARCH REPORT

**Application Number**

EP 22 15 9795

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/043600 A1 (GLOTTMANN TOMER [IL] ET AL) 6 February 2020 (2020-02-06) * paragraphs [0010] - [0081] * ----- | 1-15 | INV. G16H15/00 G06F40/00 G06T7/00 G16H50/20 |
| X | US 2020/160993 A1 (XIE YITING [US] ET AL) 21 May 2020 (2020-05-21) * paragraphs [0007] - [0061] * ----- | 1-4,7, 11-15 | |

**TECHNICAL FIELDS SEARCHED (IPC)**

G16H
G06F
G06T

The present search report has been drawn up for all claims

| Place of search | Date of completion of the search | Examiner |
|---|---|---|
| Munich | 22 July 2022 | Rivera Pons, Carlos |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

....................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 9795

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2022

| Patent document cited in search report | | Publication date | Patent family member(s) | | Publication date |
|---|---|---|---|---|---|
| US 2020043600 | A1 | 06-02-2020 | CN 112868020 | A | 28-05-2021 |
| | | | EP 3830748 | A2 | 09-06-2021 |
| | | | IL 280418 | A | 01-03-2021 |
| | | | US 2020043600 | A1 | 06-02-2020 |
| | | | US 2020321100 | A1 | 08-10-2020 |
| | | | WO 2020026033 | A2 | 06-02-2020 |
| US 2020160993 | A1 | 21-05-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KAHN JR, C.E. et al.** Toward best practices in radiology reporting. *Radiology,* 2009, vol. 252 (3), 852-856
- **POWEL, D.K. ; SILBERZWEIG, J.E.** State of structured reporting in radiology, a survey. *Academic radiology,* 2015, vol. 22 (2), 226-233
- ESR paper on structured reporting in radiology. *Insights into imaging,* 2018, vol. 9, 1-7
- **RAO et al.** Utility of artificial intelligence tool as a prospective radiology peer reviewer - Detection of unreported intracranial hemorrhage. *academic radiology,* vol. 28 (1), 85-93
- **MAHAJAN et al.** Deploying deep learning for quality control: An AI-assisted review of chest X-rays reported as normal in routine clinical practice. *Radiological Society of North America 2019 Scientific Assembly and Annual Meeting,* 01 December 2019
- Text Parsing. **PYYSALO S.** Encyclopedia of Systems Biology. Springer, 2013
- **TANG, Y.X. ; TANG, Y.B. ; PENG, Y. ; YAN, K. ; BAGHERI, M. ; REDD, B.A. ; BRANDON, C.J. ; LU, Z. ; HAN, M. ; XIAO, J.** Automated abnormality classification of chest radiographs using deep convolutional neural networks. *NPJ digital medicine,* 2020, vol. 3 (1), 1-8
- **CALLI, E. ; SOGANCIOGLU, E. ; VAN GINNEKEN, B. ; VAN LEEUWEN, K.G. ; MURPHY, K.** Deep learning for chest X-ray analysis: A survey. *Medical Image Analysis,* 2021, vol. 72, 102125
- **HANSELL, D.M. ; BANKIER, A.A. ; MACMAHON, H. ; MCLOUD, T.C. ; MULLER, N.L. ; REMY, J.** Fleischner Society: glossary of terms for thoracic imaging. *Radiology,* 2008, vol. 246 (3), 697-722
- **PALMER, W. ; BANCROFT, L. ; BONAR, F. ; CHOI, J.A. ; COTTEN, A. ; GRIFFITH, J.F. ; ROBINSON, P. ; PFIRRMANN, C.W.** Glossary of terms for musculoskeletal radiology. *Skeletal Radiology,* 2020, vol. 49 (1), 1-33
- **ATALAR, M. ; ÖZTOPRAK, B. ; GÜMÜS, C. ; EGILMEZ, H. ; BULUT, S.** Classical Signs and Appearances in Pediatric Neuroradiology. *European Congress of Radiology-ECR,* March 2014
- **SUCHANEK et al.** Advances in automated knowledge base construction. *SIGMOD records journal,* 2013
- **MARCH ; NIKLAUS, C et al.** A survey on open information extraction. *arXiv: 1806.05599,* 2018

- **ZHANG et al.** When radiology report generation meets knowledge graph. *Proceedings of the AAAI Conference on Artificial Intelligence,* April 2020, vol. 34 (07), 12910-12917
- **HOU et al.** Multi-Label Learning with Visual-Semantic Embedded Knowledge Graph for Diagnosis of Radiology Imaging. *IEEE Access,* 2021, vol. 9, 15720-15730
- **BANKO, MICHELE et al.** Open Information Extraction from the Web. *Conference on Artificial Intelligence,* 2007
- **DALVI, B et al.** IKE-an interactive tool for knowledge extraction. *proceedings of the 5th Workshop on Automated Knowledge Base Construction,* June 2016, 12-17
- **LAO et al.** Random walk inference and leaning in a large scale knowledge base. *proceedings of the 2011 conference on empirical methods in natural language processing,* July 2011, 529-539
- **WANG et al.** Programming with personalized pagerank: a locally groundable first-order probabilistic logic. *proceedings of the 22nd ACM international conference on Information & Knowledge Management,* October 2013, 2129-2138
- **PUJA et al.** Large-Scale Knowledge Graph Identification Using PSL. *AAAI Fall Symposia,* 2013
- Learning to refine an automatically extracted knowledge base using Markov logic. **JIANG, S et al.** 12th International Conference on Data Mining. IEEE, December 2012, 912-917
- **LINDBERG, C.** The Unified Medical Language System (UMLS) of the National Library of Medicine. *Journal (American Medical Record Association),* 1990, vol. 61 (5), 40-42
- **MANNING, C.D. et al.** The Stanford CoreNLP natural language processing toolkit. *Proceedings of 52nd annual meeting of the association for computational linguistics: system demonstrations,* June 2014, 55-60
- **WU, S. ; ROBERTS, K. ; DATTA, S. ; DU, J. ; JI, Z. ; SI, Y. ; SONI, S. ; WANG, Q. ; WEI, Q. ; XIANG, Y.** Deep learning in clinical natural language processing: a methodical review. *Journal of the American Medical Informatics Association,* 2020, vol. 27 (3), 457-470

- **WANG, Y. ; WANG, L. ; RASTEGAR-MOJARAD, M. ; MOON, S. ; SHEN, F. ; AFZAL, N. ; LIU, S. ; ZENG, Y. ; MEHRABI, S. ; SOHN, S.** Clinical information extraction applications: a literature review. *Journal of biomedical informatics,* 2018, vol. 77, 34-49
- **WALTL, B. ; BONCZEK, G. ; MATTHES, F.** Rule-based information extraction: Advantages, limitations, and perspectives. *Jusletter IT,* February 2018
- **CHITICARIU, L. ; LI, Y. ; REISS, F.** Rule-based information extraction is dead! long live rule-based information extraction systems!. *Proceedings of the 2013 conference on empirical methods in natural language processing,* October 2013, 827-832
- RATCHET: Medical Transformer for Chest X-ray Diagnosis and Reporting. **HOU, B. ; KAISSIS, G. ; SUMMERS, R.M. ; KAINZ, B.** International Conference on Medical Image Computing and Computer-Assisted Intervention. Springer, September 2021, 293-303
- **BANERJEE, I. ; LING, Y. ; CHEN, M.C. ; HASAN, S.A. ; LANGLOTZ, C.P. ; MORADZADEH, N. ; CHAPMAN, B. ; AMRHEIN, T. ; MONG, D. ; RUBIN, D.L.** Comparative effectiveness of convolutional neural network (CNN) and recurrent neural network (RNN) architectures for radiology text report classification. *Artificial intelligence in medicine,* 2019, vol. 97, 79-88